# EUROPEAN PATENT APPLICATION

(11) **EP 1 251 174 A1**
(43) Date of publication of application: **23.10.2002**
(21) Application number: 01900664.2
(22) Date of filing: 11.01.2001
(51) Int. Cl.: C12N 15/45, C12Q 1/68

(54) **USE OF PARAMYXOVIRUS VECTOR IN GENE TRANSFER INTO BLOOD VESSEL**

(30) Priority: 19.01.2000 JP 2000014136
(71) Applicant: Dnavec Research Inc., Ibaraki 305-0856 (JP)
(72) Inventor: MASAKI, Ichirou, Kurume-shi, Fukuoka 830-0038 (JP); YONEMITSU, Yoshikazu, Fukuoka-shi, Fukuoka 813-0043 (JP); SUEISHI, Katsuo, Fukuoka-shi, Fukuoka 815-0073 (JP); HASEGAWA, Mamoru Dnavec Research Inc., Tsukuba-shi, Ibaraki 305-0856 (JP); KINOH, Hiroaki Dnavec Research Inc., Tsukuba-shi, Ibaraki 305-0856 (JP)
(74) Representative: Ahner, Francis
(86) International application number: JP0100087
(87) International publication number: WO01053491

(57) **Abstract**

Use of a recombinant Paramyxovirus vector has enabled transferring nucleic acid into the blood vessel at high efficiency by short exposure. The present invention provides a Paramyxovirus vector used for transferring nucleic acid into the vascular cells and a method for transferring nucleic acid using such vector. Pretreatment of blood vessels with protease significantly improved the efficiency of transfection into the medial layer. The expression of transfected genes was stable in the vascular cells for a long period. The method of the present invention enables efficiently transferring genes into tissues such as the blood vessel lumen, media, and adventitia in short time in such applications as gene therapy.

SEQUENCE LISTING

<110> DNAVEC Research Inc.

<120> USE OF PARAMYXOVIRUS VECTOR FOR GENE TRANSFER INTO BLOOD VESSEL

<130> D3-106PCT

<140>
<141>

<150> JP 2000-014136
<151> 2000-01-19

<160> 8

<170> PatentIn Ver. 2.0

<210> 1
<211> 10
<212> DNA
<213> Artificial Sequence

<220>
<223> Description of Artificial Sequence: Artificially
      Synthesized Sequence

<400> 1
ctttcaccct                                                        10

<210> 2
<211> 15
<212> DNA
<213> Artificial Sequence

<220>
<223> Description of Artificial Sequence: Artificially
      Synthesized Sequence

<400> 2
tttttcttac tacgg                                                  15

<210> 3
<211> 18
<212> DNA
<213> Artificial Sequence

<220>
<223> Description of Artificial Sequence: Artificially
      Synthesized Sequence

<400> 3
cggccgcaga tcttcacg                                                    18

<210> 4
<211> 18
<212> DNA
<213> Artificial Sequence

<220>
<223> Description of Artificial Sequence: Artificially
      Synthesized Sequence

<400> 4
atgcatgccg gcagatga                                                    18

<210> 5
<211> 18
<212> DNA
<213> Artificial Sequence

<220>
<223> Description of Artificial Sequence: Artificially
      Synthesized Primer Sequence

<400> 5
gttgagtact gcaagagc                                                    18

<210> 6
<211> 42
<212> DNA
<213> Artificial Sequence

<220>
<223> Description of Artificial Sequence: Artificially
      Synthesized Primer Sequence

<400> 6
tttgccggca tgcatgtttc ccaaggggag agttttgcaa cc                    42

<210> 7
<211> 18
<212> DNA
<213> Artificial Sequence

<220>
<223> Description of Artificial Sequence: Artificially
      Synthesized Primer Sequence

<400> 7
atgcatgccg gcagatga                                               18

<210> 8
<211> 21
<212> DNA
<213> Artificial Sequence

<220>
<223> Description of Artificial Sequence: Artificially
      Synthesized Primer Sequence

<400> 8
tgggtgaatg agagaatcag c                                           21

## Description

### Technical Field

The present invention relates to a use of a Paramyxovirus vector for gene transfer into the blood vessel.

### Background Art

Since the successful transfer of a recombinant gene into the vessel wall *in vivo* (Nabel E.G. et al., Science 244:1342-1344 (1989) ; Nabel E.G. et al., Science 249:1285-1288 (1990)), gene therapy has gained huge expectations as a technology for the treatment of human vascular diseases, and thereby, numbers of vector systems have been developed and tested. Although the studies in animals were successful, there are several critical limitations on the vector systems including gene transfer efficiency, besides safety concerns. Thus, it is yet difficult to apply the results of those studies to clinical trials (DeYoung M.B. et al., Circ. Res. 82:306-313 (1998)).

For instance, in PTCA (percutaneous transluminal coronary angioplasty), constriction is released by inflating a balloon at stenotic site. However, 30 to 40% of the balloon-treated vessels develop restenosis, which is a big clinical issue. The primary cause of restenosis is the growth of neointima and the reconstruction of the blood vessel. Neointima, invaded by macrophages and enlarged by atherosclerosis, is mainly composed of the vascular smooth muscle cells migrating from tunica media, and the extracellular matrix. For gene therapies to block restenosis or to treat arteriosclerosis, it is effective to transfer genes into the artery; however, using the existing vectors or liposomes, the transfer efficiency is low. Macrophages and vascular smooth muscle cells are the targets of treatment. Macrophages are located in intima under basal lamina, and vascular smooth muscle cells are located in vascular tunica media. Barriers of vascular endothelial cells and basal lamina exist in the gene transfer into macrophages, and a barrier of elastic fibers in addition to the two exists in the gene transfer into smooth muscle cells.

Recombinant adenovirus vector, one of the most widely used vectors for gene transfer into the blood vessel, is known for its relatively high transfection efficiency. However, several problems remain to be solved before application to patients. Although recombinant adenovirus vector is capable of transferring a foreign gene effectively into intact endothelial cells (ECs) and adventitia in animals (Rome J.J. et al., Arterioscler. Thromb. 14:148-161 (1994) ; Steg P.G. et al., Circulation 90:1648-1656 (1994)) and in humans as well (Rekhter M.D. et al., Circ. Res. 82:1243-1252 (1998)), the efficiency of adenovirus incorporation into the atherosclerotic region and medial layer is relatively low in rabbits (Feldman L.J. et al., J. Clin. Invest. 95:2662-2671 (1995)) and humans (Rekhter M.D. et al., Circ. Res. 82:1243-1252 (1998)). Thereby, the effectiveness of adenovirus is limited in gene therapies targeted at quiescent cells. In fact, administration of adenovirus from the interior of the blood vessel only leads to the restricted infection of endothelial cells, leaving macrophages and smooth muscle cells uninfected. Thus, it is yet difficult to infect a gene for treatment directly into the cells causing such diseases as atherosclerosis. More importantly, the gene transfer using adenovirus requires long exposure time to obtain maximal transfection efficiency (Feldman L.J. et al., J. Clin. Invest. 95:2662-2671 (1995)). This is thought to be because intracellular incorporation of vector particles through Coxsackie-adenovirus receptor (CAR) requires long-term contact. For instance, in PTCA, coronary arterial blood flow can be blocked at most for several minutes. Thus, it is a big obstacle for the clinical application of adenovirus vector.

To solve the above problems associated with use of adenovirus vector, several different approaches have been attempted. In the first method, the infectivity is improved by ablating the endothelial cell using the balloon catheter. In the second method, the endothelial cell is first ablated with the balloon catheter, and then infection is performed while pressure is applied. In the last method, after ablation of endothelial cells with the balloon catheter, the tissue surface is digested with elastase. None of the methods, however, achieve sufficient infectivity; moreover, each of infection and pressure application takes about 30 minutes or more to be completed (Guzman R.J. et al., Circulation 88:2838-2848 (1993); Maillard L. et al., Gene Ther. 5:1023-1030 (1998); Rekhter M.D. et al., Circ. Res. 82:1243-1252 (1998) ; Rome J.J. et al., Hum. Gene Ther. 5:1249-1258 (1994); Weeks B.S. et al., Arch. Virol. 145:385-396 (2000)).

A liposome coated with the envelope protein of Sendai virus (SeV, or hemagglutinating virus of Japan; HVJ), so called HVJ-liposome is capable of transferring genes at relatively high efficiency. The present inventors already reported that *in vivo* injection of HVJ-liposome into rabbit carotid artery at 150 mmHg for 10 minutes resulted in transfection in the medial smooth muscle cell at efficiency of 80% or more (Yonemitsu Y. et al., Lab. Invest. 75:313-323 (1996)). Examination of the blood vessel by electron microscopy revealed that the vector particles penetrate to all over the medial layer, suggesting that the permeability of the blood vessel walls of middle-sized animals is relatively high. The present inventors applied the vector system to induce vascular disorders *in vivo* by overexpressing several genes including vascular endothelial cell growth factor (VEGF) and human cytomegarovirus immediate early gene (Yonemitsu Y. et al., Lab. Invest. 75:313-323 (1996) ; Yonemitsu Y. et al., Biochem. Biophys. Res. Commun. 231:447-451 (1997)), and also to suppress angiogenic diseases *in vivo* using endothelial constitutive NO synthetase gene (Matsumoto T. et al., J. Vasc. Surg. 27:135-144 (1998)) or wild-type p53 gene (Yonemitsu Y. et al., Circ. Res. 82:147-156 (1998)). However, the results of preclinical tests and preliminary experiments using diseased human saphenous veins and coronary arteries done by the present inventors showed that the efficiency of gene transfer and expression level achieved by the vector system were insufficient as previously reported in adenovirus (Feldman L.J. et al., J. Clin. Invest. 95:2662-2671 (1995)). Specifically, transfection using HVJ-liposome into neointima and media is restricted in the only a few layers from the surface even at 1 atmospheric pressure, which suggests that the permeability of vector particles is lost. These results obtained by using adenovirus or HVJ-liposome indicate that there exist biological barriers in diseased human vessel walls. Therefore, the development of a new vector system that can overcome such problems has been desired.

### Disclosure of the Invention

An object of the present invention is to provide a virus vector that is capable of transferring genes into the vascular cells with short exposure and furthermore expressing thus transferred genes therein constitutively, and its use. More specifically, the present invention provides a Paramyxovirus vector for gene transfer into the vascular cells and a method of gene transfer into the vascular cells using such vector.

The present inventors utilized reverse genetics technology of Sendai virus (abbreviated to "SeV"), and succeeded in the development of a recombinant SeV vector for gene transfer (Kato A. et al., Genes Cells 1:569-579 (1996); Kato A. et al., EMBO J. 16:578-587 (1997); Yu D. et al., Genes Cells 2:457-466 (1997); Hasan M.K. et al., J. Gen. Virol. 78:2813-2820 (1997) ; SakaiY. et al., FEBSLett. 456:221-226 (1999); WO 97/16538 and WO 97/16539).

The present inventors characterized the gene transfer into the blood vessel using the recombinant SeV vector, and examined whether the vector could overcome the problems shared by the existing gene transfer vectors. Specifically, the present inventors carried out (1) the evaluation of the transfection activity for SeV *in vitro,* including the transfection efficiency, gene expression efficiency, persistence of gene expression, and effect of vector exposure time, from clinical point of view; (2) the examination of the transfection ability of SeV into human blood vessel and the cell types to be transfected, using diseased human great saphenous vein obtained surgically from patients having varix in the lower limb.

The results showed that recombinant SeV containing the firefly luciferase gene achieved constitutive gene expression in a dose dependent manner in both proliferating and quiescent cells , suggesting that the genome was stable over one month. In addition, it was revealed that nearly maximal level of gene expression was obtained by short exposure in both cultured cells and human vein. The result of ex vivo experiment using human diseased great saphenous vein and SeV encoding nuclear-targeted lacZ gene showed that the endothelial cells (EC) in the lumen and vasa vasora (vessel wall nutrient vessel) and adventitial fibroblasts were transfected at high efficiency by both transfection into the lumen and simple floating method.

Thus, the present inventors found that (1) short exposure of solution containing the Paramyxovirus vector to the blood vessel was sufficient to perform gene transfer efficiently, and (2) both virus genome and the expression of foreign gene were stable for a long time and were, at least *in vitro,* relatively stable for one month or more in both proliferating cells and quiescent cells at least *in vitro.* These results obviously contrast with those obtained with adenovirus vector, which has been used as a promising standard in the field of gene transfer into the blood vessel. Adenovirus vector has been shown to have several advantages such as high expression level of transferred gene and high efficiency of gene transfer, both of which are independent of cell cycle. These features are shared by the Paramyxovirus vector. Taking the features of the Paramyxovirus vector into account, it is possible to establish gene therapies targeted at the blood vessel for individual clinical applications.

When the infectivity of lacZ/Adenovirus vector and that of lacZ-SeV were compared with each other in gene transfer into in vitro organ culture using rat thoracic artery, lacZ-SeV achieved high infectivity in vascular endothelial cells at lower moi with short exposure (2 minutes). Thus, it became clear that the Paramyxovirus vector of the present invention can overcome the temporal limitation that is one of the big problems for infection with adenovirus vector.

The present inventors further found that digesting the extracellular matrix such as basal lamina and elastic fibers by treating with protease before administration of vector enabled transfection into media at high efficiency. Improved efficiency of transfection into vascular media by protease treatment was observed when protease and vector of the present invention were mixed and then administered simultaneously, as well as when the vector was administered after protease treatment. In particular, the finding that simultaneous administration of premixed protease and vector achieved transfection into the medial layer of blood vessel was unpredictable. Thus, the present inventors first demonstrated that it is possible to perform protease treatment and vector infection simultaneously. Examination of the effect of pretreatment with various proteases on the infectivity of lacZ-SeV using rat thoracic artery that were organ cultured *in vitro* revealed that all the examined protease improved the infectivity of lacZ-SeV. In particular, pretreatment with collagenase, tPA, and MMP9 was markedly more effective than that with elastase, which has been shown to be effective in adenovirus vector infection. Examination of sections which were constructed to determine the regions containing cells infected by SeV vector revealed that pretreatment with collagenase, tPA, or MMP9 resulted in infection even into the smooth muscle cells of media beyond the vascular endothelial cells and basal lamina. *In vivo* gene transfer experiments using rat abdominal aorta revealed, as *in vitro* experiments, that pretreatment with collagenase, tPA, and MMP9 resulted in infection in media. In addition, the time required for performing a series of protease treatment and infection could be shortened to 7 to 9 minutes or shorter (for instance no more than 5 minutes) . Thus, the vector of the present invention has enabled gene transfer into cells in media, which has been unreachable by conventional vectors, and thereby it solved not only temporal but also spatial limitations on gene transfer into blood vessels.

It has been reported that infectivity of adenovirus vector was improved by ablation of endothelial cells using the balloon catheter and/or digestion with elastase before vector administration (Maillard L. et al., Gene Ther. 5:1023-1030 (1998)). However, the infectivity was still low, especially in the smooth muscle cells. In contrast, the infectivity of the vector of the present invention is high, and the types of target cells are also expanded. Combining protease treatment with the Paramyxovirus vector of the invention which only requires short exposure to infect and, it is also possible to more efficiently transfer genes into macrophages and smooth muscle cells in the blood vessel.

Thus, the present invention relates to a Paramyxovirus vector that is used for gene transfer into the blood vessel, and a method for transferring a gene using such vector. More specifically, the present invention relates to:
(1) a method for transferring a nucleic acid into a vascular cell, said method comprising a step of contacting a recombinant Paramyxovirus vector or a cell comprising said vector with said vascular cell;
(2) a method for transferring a nucleic acid into a vascular cell, said method comprising performing the following steps (a) and (b) sequentially or simultaneously:
   (a) treating a tissue comprising a vascular cell with a protease, and
   (b) contacting a recombinant Paramyxovirus vector or a cell comprising said vector with said vascular cell;
(3) the method according to (2), wherein said protease is selected from the group consisting of collagenase, urokinase, elastase, tissue plasminogen activator, plasmin, and matrix metalloproteinases;
(4) the method according to any one of (1) to (3), wherein said nucleic acid contained in the recombinant Paramyxovirus vector comprises a foreign gene;
(5) the method according to any one of (1) to (4), wherein said vascular cell is selected from the group consisting of an endothelial cell in a vascular lumen, an endothelial cell in a vasa vasorum (vessel wall nutrient vessel) , a vascular smooth muscle cell in vascular media, and an adventitial cell;
(6) the method according to any one of (1) to (5), wherein said Paramyxovirus is Sendai virus;
(7) a recombinant Paramyxovirus vector that transfers a nucleic acid into a vascular cell;
(8) the vector according to (7) , wherein said Paramyxovirus is Sendai virus;
(9) the vector according to (7) or (8), wherein said nucleic acid contained in the recombinant Paramyxovirus vector comprises a foreign gene;
(10) the vector according to any one of (7) to (9), wherein said vascular cell is selected from the group consisting of an endothelial cell in a vascular lumen, an endothelial cell in a vasa vasorum (vessel wall nutrient vessel) , a vascular smooth muscle cell in vascular media, and an adventitial cell;
(11) a composition for gene transfer into a vascular cell, said composition comprising the recombinant Paramyxovirus vector according to any one of (7) to (10);
(12) a kit for gene transfer into a vascular cell, said kit comprising the recombinant Paramyxovirus vector according to any one of (7) to
(10) and a protease; and
(13) the kit according to (12), wherein said protease is selected from the group consisting of collagenase, urokinase, elastase, tissue plasminogen activator, plasmin, and matrix metalloproteinases.

Herein, a "Paramyxovirus vector" is defined as a vector (or carrier) that is derived from the Paramyxovirus and that is used for gene transfer to host cells. The Paramyxovirus vector of the present invention may be RNP or a virus particle having infectivity. Here, "infectivity" is defined as the ability of the Paramyxovirus vector to transfer, through its cell adhesion and membrane fusion abilities, a gene contained in the vector to cells to which the vector is adhered. In a preferred embodiment, the Paramyxovirus vector of the present invention carries a foreign gene in an expressible manner. The Paramyxovirus vector may have a replication ability, or may be a defective vector without the replication ability. Herein, "replication ability" is defined as the ability of virus vectors to replicate and produce infective virus particles in host cells infected with the virus vectors.

Herein, a "recombinant" Paramyxovirus vector is defined as that constructed by gene engineering or its amplified products. For instance, recombinant Paramyxovirus vectors can be generated by reconstitution of a recombinant Paramyxovirus cDNA.

Herein, a Paramyxovirus is defined as a virus of the *Paramyxoviridae* family or a derivative thereof. The present invention can be applied to, for example, Paramyxoviruses such as Sendai virus, Newcastle disease virus, Mumps virus, Measles virus, Respiratory syncytial virus, rinderpest virus, distemper virus, simian parainfluenza virus (SV5), type I, II, and III human parainfluenza virus of the *Paramyxoviridae*. The virus of the present invention may be preferably a virus of the genus *Paramyxovirus* or a derivative thereof . Viruses of the genus *Paramyxovirus* to which the present invention is applicable include type I parainfluenza viruses including Sendai virus and human HA2, type II parainfluenza viruses including simian SV5 and SV41 and human CA, type III parainfluenza viruses including bovine SF and human HA1, type IV parainfluenza viruses including subtype A and subtype B, Mumps virus, Newcastle disease virus, and many other viruses of the genus *Paramyxovirus.* Most preferably, the Paramyxovirus of the invention may be the Sendai virus. These viruses may be wild-type strains, mutant strains, laboratory-passaged strains, artificially constructed strains, or so on. Incomplete viruses such as the DI particle (Willenbrink W. and Neubert W. J., J. Virol., 1994, 68, 8413-8417), synthesized oligonucleotides, and so on, may also be utilized as material for generating the virus vector of the present invention.

Genes encoding proteins of a Paramyxovirus include NP, P, M, F, HN, and L genes. Here, the "NP, P, M, F, HN, and L genes" represent those encoding the nucleocapsid protein, phosphoprotein, matrix protein, fusion protein, hemagglutinin-neuraminidase, and large protein, respectively. Genes of each virus of the subfamily Paramyxovirus are described generally as follows. In general, NP gene may also be indicated as "N gene".

| | | | | | | | |
|---|---|---|---|---|---|---|---|
| Paramyxovirus | NP | P/C/V | M | F | HN | - | L |
| Rublavirus | NP | P/V | M | F | HN | (SH) | L |
| Morbillivirus | NP | P/C/V | M | F | H | - | L |

For instance, the accession numbers in the nucleotide sequence database of each gene of the Sendai virus classified as a Respirovirus of *Paramyxoviridae*, are M29343, M30202, M30203, M30204, M51331, M55565, M69046, and X17218 for NP gene; M30202, M30203, M30204, M55565, M69046, X00583, X17007, and X17008 for P gene; D11446, K02742, M30202, M30203, M30204, M69046, U31956, X00584, X53056 for M gene; D00152, D11446, D17334, D17335, M30202, M30203, M30204, M69046, X00152, and X02131 for F gene; D26475, M12397, M30202, M30203, M30204, M69046, X00586, X02808, X56131 for HN gene; and D00053, M30202, M30203, M30204, M69040, X00587, and X58886 for L gene.

Here, a "gene" is defined as a genetic substance, which includes nucleic acids such as RNA and DNA. Genes may have naturally derived or artificially designed sequences. An artificially designed protein, for instance, may be a fusion protein with another protein, a dominant negative protein, including a soluble form of a receptor or a membrane-bound dominant negative receptor, a deletion form of a cell adhesion molecule, and a soluble form of a cell surface molecule. Herein, "DNA" includes single stranded DNA or a double stranded DNA.

Herein, "vascular cells" are defined as cells composing the blood vessel, and include such cells as those in the endothelium (lumen), media, adventitia, and vasa vasora. These cells include endothelial cells, smooth muscle cells, fibroblasts, etc.

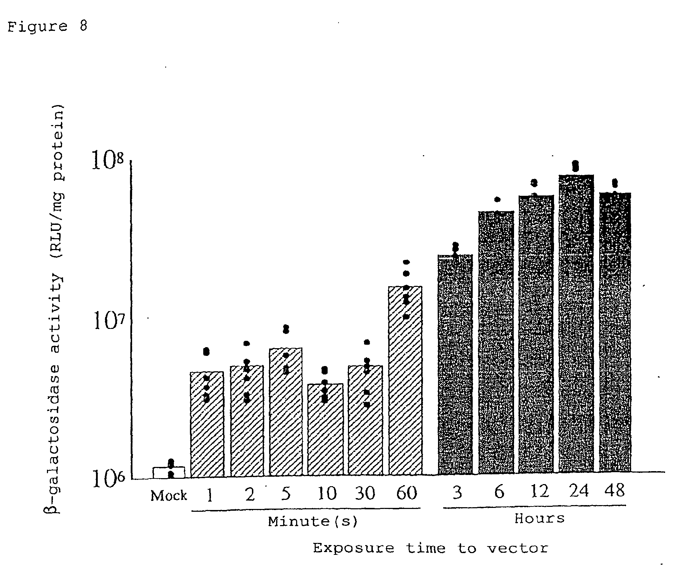

The present invention provides a use of a recombinant Paramyxovirus vector for gene transfer into the vascular cells. The present inventors have found that Sendai virus is capable of transferring genes into vascular cells with short exposure. In the experiments by the present inventors, significant gene expression was detected in vascular cells only 1 hr after the gene transfer, and the expression level increased logarithmically in a time dependent manner. Moreover, the vector of the present invention exhibited sufficient infectivity into vascular cells even with extremely short exposure (several minutes). This brings great advantage in gene transfer into the arterial lumen at the regions of PTCA, for instance, because use of a recombinant Paramyxovirus can minimize the time in which blood flow is blocked. *In vitro* studies by Guzman et al. showed that adenovirus vector containing lacZ can be transfected into only 25% of vascular smooth muscle cells (VSMC) with 5 min exposure, and 80% even with 120 min exposure (Guzman R.J. et al., Circulation 88:2838-2848 (1993)). Similar results were representatively observed in the experiments by the present inventors using BSMC (Figure 8). It suggests that this practical problem in application for PTCA can be solved by using a recombinant Paramyxovirus in gene transfer targeted at the endothelial cells.

In addition, the present inventors revealed that the genes transferred into vascular cells using a recombinant Sendai virus were persistently expressed at least over one month. This brings an advantage that continuous effect on treatment can be obtained when gene therapies targeted at vascular cells was performed using a recombinant Paramyxovirus vector.

Sendai virus vectors can be preferably utilized in clinical trials of human gene therapy in terms of safety as well. First, it is a major obstacle in high efficient gene transfer that transfected DNA must be transported into the nucleus for the expression of a foreign gene. In the case of Sendai virus and such, however, expression of a foreign gene is driven by both cellular tubulin and its RNA polymerase (L protein) in the cytoplasm. This suggests that the Sendai virus does not interact with the genome of host cells , which avoids safety problems such as tumorigenesis. Second, the Sendai virus is known to be pathogenic in rodents causing pneumonia, but not in humans, which is supported by studies showing that the intranasal administration of the wild type Sendai virus does not do harm in nonhuman primates (Hurwitz J. L. et al., Vaccine, 1997, 15, 533-540). These features suggest that Sendai virus vector can be utilized in human therapy, and further, support the notion that Sendai virus can be one of the promising alternatives in gene therapy to vascular wall.

Thus, the finding that Paramyxovirus vector has various advantages in gene transfer into vascular cells may bring great advance in gene therapy, especially those targeted at vascular cells, where the finding was obtained by the present invention. Therefore, the present invention relates to a recombinant Paramyxovirus vector used for transferring nucleic acid into a vascular cell, and a method for transferring nucleic acid into a vascular cell using such vector. The present invention also provides a use of a recombinant Paramyxovirus vector for transferring nucleic acid into vascular cells.

The recombinant Paramyxovirus vector of the present invention used for gene transfer into vascular cells is not limited to any special kind. For instance, vectors that have the replication ability and that are capable of autonomous propagation may be preferably utilized. In general, the genome of the wild type Paramyxovirus contains a short 3' leader region followed by six genes encoding N (nucleocapsid), P (phospho), M (matrix) , F (fusion), HN (hemagglutinin-neuraminidase), and L (large) proteins, and has a short 5' trailer region on the other terminus. The vector of the present invention that is able to replicate autonomously can be obtained by designing a genome having a similar structure to that described above. In addition, avector for expressing a foreign gene can be obtained by inserting the foreign gene to the genome of the above vector. The Paramyxovirus vector of the invention may have an altered alignment of virus genes, compared with wild type virus.

The Paramyxovirus vector of the invention may have deletion(s) of some of the genes that are contained in the wild type virus. For instance, in the case of the reconstitution of the Sendai virus vector, proteins encoded by NP, P/C, and L genes are thought to be required in trans, but the genes may not be a component of the virus vector. In one embodiment, an expression vector carrying genes encoding the proteins may be co-transfected into host cells with another expression vector encoding the vector genome to reconstitute a virus vector. Alternatively, an expression vector encoding the virus genome is transfected into host cells carrying genes encoding the proteins, and thus a virus vector can be reconstituted by using the proteins provided by the host cell. The amino acid sequence of these proteins may not be identical to those derived from the original virus as long as it has an equivalent or higher activity in nucleic acid transfer, and may be mutated or replaced with that of a homologous gene of another virus.

Proteins encoded by M, F, and HN genes are thought to be essential for cell-to-cell propagation of a Paramyxovirus vector. However, these proteins are not required when the vector is prepared as RNP. If genes M, F, and HN are components of the genome contained in RNP, products of these genes are produced when introduced into host cells, and virus particles having infectivity are generated. RNP vectors that produce an infective virus include a virus genome RNA encoding N, P, M, F, HN, and L genes and RNP containing N, P, and L proteins. When such RNP is introduced into cells, virus genome is expressed and replicated through functions of the proteins, and thus infective virus vectors are amplified.

RNP can be introduced into cells as a complex formed with lipofectamine, polycationic liposome, and the like. Specifically, a variety of transfection reagents can be used, for instance, DOTMA (Boehringer), Superfect (QIAGEN #301305), DOTAP, DOPE, DOSPER (Boehringer #1811169). Chloroquine may be added to prevent degradation in the endosome (Calos M. P., Proc. Natl. Acad. Sci. USA, 1983, 80, 3015). In the case of replicative viruses, the produced viruses can be amplified or passaged by re-infecting into cultured cells, chicken eggs, or animals (e.g. mammalian such as mice).

Contrastingly, the Paramyxovirus vector of the present invention may be those lacking the M, F, and/or HN genes. These vectors can be reconstituted by providing deleted gene products exogenously. Such vectors can still adhere to host cells and induce cell fusion as the wild type. However, daughter virus particles do not have the same infectivity as the original ones because the vector genome introduced into cells lacks one of the above genes. Therefore, these vectors can be useful as safe virus vectors that are capable of only a single gene transfer. For instance, genes deleted from the genome may be F and/or HN genes. Virus vectors can be reconstituted by co-transfection of an expression plasmid encoding the genome of a recombinant Paramyxovirus lacking the F gene, an expression vector for the F protein, and that for NP, P/C, and L proteins into host cells (PCT/JP00/03194 and PCT/JP00/03195). Alternatively, host cells in which the F gene is integrated into the chromosome may be used. The amino acid sequence of these proteins provided exogenously may not be identical to those of the wild type and may be mutated or replaced by a homologous protein of another virus as long as they provide equivalent or higher gene transfer activity.

The envelope protein of the Paramyxovirus vector of the invention may contain another protein than the envelope protein of the original vector genome. There is no limitation on such proteins. These may include envelope proteins of other viruses such as the G protein of the vesicular stomatitis virus (VSV-G). Thus, the Paramyxovirus vector of the invention includes a pseudo type virus vector that has an envelope protein derived from a virus different from the original virus. F protein and/or HN protein originally contained in Paramyxovirus may be deleted.

Incomplete virus such as DI particles (J. Virol. 68:8413-8417 (1994)) or synthetic oligonucleotides may be used as a component of the vector of the present invention instead of complete Sendai virus genome.

Also, the vector of the invention may contain, on the surface of its envelope, adhesion molecules, ligands, receptors, or fragments of these targeted at particular cells . For example, a chimeric protein having a polypeptide derived from adhesion molecules, ligands, or receptors targeted at particular cells in its extracellular domain and a polypeptide derived from the virus envelope protein in its intracellular domain can be used as an envelope. It enables the production of a vector targeting a particular tissue. These proteins may be encoded by the virus genome itself, or supplied at the time of virus reconstitution through expression of genes other than virus genome (for example, another expression vector or host cell chromosome).

The virus genes contained in the vector of the present invention may be altered, for example, to reduce antigenicity or enhance RNA transcription efficiency or replication efficiency. Specifically, it is possible to alter at least one of the NP, P/C, and L genes, which are genes of replication factors, to enhance transcription or replication. It is also possible to alter the HN protein, a structural protein having hemagglutinin activity and neuraminidase activity, to enhance the virus stability in blood by weakening the former activity and to regulate infectivity by altering the latter activity. It is also possible to alter the F protein, which is implicated in membrane fusion, to regulate the fusion ability of membrane-fused liposomes. Furthermore, it is possible to generate a virus vector that is engineered to have weak antigenicity through analyzing the antigen presenting epitopes and such of possible antigenic molecules on the cell surface such as the F protein and HN protein.

The virus vector of the present invention may contain a foreign gene in the genome RNA. A recombinant Paramyxovirus vector containing a foreign gene can be obtained by inserting the gene into the genome of the above-described Paramyxovirus vector. The foreign gene may be a gene encoding a protein to be expressed in target vascular cells but is not limited thereto. It may encode a natural protein, or an altered protein having a deletion, substitution, or insertion as long as the protein has a function equivalent to that of the natural protein . Alternatively, it may be an artificially designed protein such as a dominant negative mutant. Also, it may be a nucleic acid that does not encode a protein, such as antisense or ribozyme.

For instance, for the purpose of gene therapy and such, a gene used to treat a target disease may be inserted into the DNA encoding the genome of the virus vector (the virus vector DNA). In the case of inserting a foreign gene into Sendai virus vector DNA, a sequence comprising nucleotides of multiples of six is desirably inserted between the transcription end sequence (E) and the transcription start sequence (S) (Calain P. and Roux L., J. Virol., 1993, 67 (8), 4822-4830). A foreign gene can be inserted upstream and/or downstream of each of the virus genes (NP, P, M, F, HN, and L genes). In order not to interfere with the expression of upstream and downstream genes, an E-I-S sequence (transcription end sequence-intervening sequence-transcription start sequence) or a portion of it may be suitably placed upstream or downstream of a foreign gene so that E-I-S sequence is located between each gene.

Expression level of inserted foreign genes can be regulated by the type of transcription start sequence that is attached to the upstream of the genes (PCT/JP00/06051). It also can be regulated by the position of insertion and the sequence surrounding the gene. In the Sendai virus, for instance, the closer to the 3'-terminus of the negative strand RNA of the virus genome (the closer to NP gene in the gene arrangement on the wild type virus genome) the insertion position is, the higher the expression level of the inserted gene will be. To achieve a high expression of a foreign gene, it is preferably inserted into the upstream region of the negative stranded genome such as the upstream of the NP gene (3'flanking sequence on the minus strand), or between NP and P genes. Conversely, the closer to the 5'-terminus of the negative strand RNA (the closer to L gene in the gene arrangement on the wild type virus genome) the insertion position is, the lower the expression level of the inserted gene will be. To reduce the expression of a foreign gene, it may be inserted into the most 5' position on the negative strand, that is, downstream of the L gene in the wild type virus genome (5' flanking region of the L gene on the negative strand) or upstream of the L gene (3' flanking region of L gene on the negative strand) (Japanese Patent Application No. 2000-152726). Thus, the insertion position of a foreign gene can be properly adjusted so as to obtain a desired expression level of the gene or optimize the combination of the insert with the virus genes surrounding it. For instance, if the overexpression of a gene introduced by a high titer virus vector may cause toxicity, it is possible not only to control the virus titer, but also to reduce the expression level of individual vectors by designing the insertion position closer to the 5' -terminus of the negative strand, or replacing the transcription start sequence with one having lower efficiency so as to obtain an appropriate therapeutic effect.

To help the easy insertion of a foreign gene, a cloning site may be designed at the position of insertion. For example, the cloning site may be the recognition sequence of restriction enzymes. The restriction sites in the virus vector DNA can be used to insert a foreign gene . The cloning site may be a multicloning site that contains recognition sequences for multiple restriction enzymes. The vector of the present invention may have other foreign genes at positions other than that used for above insertion.

Construction of a recombinant Sendai virus vector having a foreign gene can be performed as follows, for example, according to the method described (Kato A. et al., EMBO J., 1997, 16, 578-587; Yu D. et al., Genes Cells, 1997, 2, 457-466).

First, a DNA sample containing a cDNA sequence encoding a desired foreign gene is prepared. It is preferable that the concentration of the sample is 25 ng/µl or higher and that it can be detected as a single plasmid by electrophoresis. The following description is an example where a foreign gene is inserted into the NotI site of virus genome DNA. If the cDNA sequence contains a NotI site, the site is desirably removed in advance by altering the nucleotide sequence using site-directed mutagenesis and such while maintaining the encoded amino acid sequence. A desired DNA fragment is amplified by PCR from the DNA sample. In order to obtain a fragment having NotI sites at both ends and to add a single copy of the transcription end sequence (E), intervening sequence (I), and transcription start sequence (S) of the Sendai virus (EIS sequence) to one end, a synthesized DNA primer pair, namely, a pair of a forward primer (sense strand) comprising a part of the desired gene, and a reverse primer (antisense) comprising a NotI recognition site, E, I, and S sequences, and part of the desired gene, is prepared.

For example, the forward synthetic DNA sequence contains two or more nucleotides at the 5'-terminus to insure digestion with NotI (preferably 4 nucleotides not containing a sequence derived from the NotI recognition site, such as GCG and GCC; more preferably ACTT). To the 3'-terminus of the sequence, the NotI recognition sequence GCGGCCGC is added. Furthermore, to the 3'-terminus, as a spacer, any 9 nucleotides or those of 9 plus multiples of 6 are added. Furthermore, to the 3'-terminus, a sequence of approximately 25 nucleotides corresponding to the ORF of the desired cDNA starting from the initiation codon ATG is added. The 3'-terminus of the forward synthetic oligo DNA containing approximately 25 nucleotides of the desired cDNA is preferably selected so that the last nucleotide is G or C.

The reverse synthetic DNA sequence contains two or more nucleotides at the 5'-terminus (preferably 4 nucleotides not containing a sequence derived from the NotI recognition site, such as GCG and GCC; more preferably ACTT). To the 3'-terminus of the sequence, the NotI recognition sequence GCGGCCGC is added. Furthermore, to the 3'-terminus, a spacer oligo DNA is added to adjust the length of the primer. The length of the oligo DNA is designed so that it is a multiple of 6 nucleotides including the NotI recognition sequence GCGGCCGC, the sequence complementary to the cDNA, and the EIS sequence derived from the Sendai virus genome as described below (so-called "rule of six"; Kolakofski D. et al., J. Virol., 1998, 72, 891-899; Calain P. and Roux L., J. Virol., 1993, 67, 4822-4830). Furthermore, to the 3'-terminus of the added sequence, complementary sequences to the S sequence of the Sendai virus, preferably 5'-CTTTCACCCT-3' (SEQ ID NO: 1), to the I sequence, preferably 5'-AAG-3', and to the E sequence, preferably 5'-TTTTTCTTACTACGG-3' (SEQ ID NO: 2) are added. Finally, to the 3'-terminus, a sequence, which is selected so that the last nucleotide of the complementary sequence of the desired cDNA becomes G or C, is added, where the last nucleotide is approximately 25 nucleotides upstream from the termination codon. Thus, the 3'-teminus of the reverse synthetic oligo DNA is prepared.

PCR can be performed by a common method using, for example, ExTaq polymerase (TaKaRa). Vent polymerase (NEB) may be used preferably, and the amplified fragment is digested with NotI, and inserted into the NotI site of the plasmid vector pBluescript. The nucleotide sequence of the obtained PCR product is checked with an automated DNA sequencer, and a plasmid having the correct sequence is selected. The insert is excised from the plasmid by NotI digestion, and subcloned into the NotI site of the plasmid (for example, pSeV18⁺b(+) (Yu, D. et al., Genes to Cells 2: 457-466, 1997) or pSeV(+) (Kato, A. et al., EMBO J. 16: 578-587, 1997)) containing the genomic cDNA to obtain recombinant Sendai virus cDNA into which the foreign cDNA is inserted. Alternatively, the PCR products may be directly cloned into the NotI site of the latter plasmid to obtain recombinant Sendai virus cDNA.

For example, recombinant Sendai virus genomic cDNA can be constructed according to the methods in the literature (Kato A. et al., EMBO J., 1997, 16, 578-598; Hasan M. K. et al., J. Gen. Virol., 1997, 78, 2813-2820). Specifically, a spacer sequence of 18 bp containing the NotI site (5'-(G)-CGGCCGCAGATCTTCACG-3'; SEQ ID NO: 3) is inserted into an adjacent gene locus of a cloned Sendai virus genomic cDNA (pSeV(+)) between the leader sequence and the 5' -terminus of a sequence encoding the N protein, and the plasmid pSeV18⁺b(+) containing a self-cleavable ribozyme site derived from the antigenomic strand of the hepatitis delta virus is obtained (Hasan M. K. et al., J. General Virol., 1997, 78, 2813-2820). A foreign gene fragment is inserted into the NotI site of pSeV18⁺b(+) to obtain a recombinant Sendai virus cDNA into which a desired foreign gene has been inserted.

Thus constructed recombinant Paramyxovirus vector DNA is transcribed *in vitro* or in cells, and RNP is reconstituted in the presence of L, P, and NP proteins to generate a virus vector comprising the RNP. The present invention provides a method for producing the Paramyxovirus vector of the invention, the method comprising transcribing a DNA encoding the genome of the virus. It also provides a DNA for producing the Paramyxovirus vector of the invention, the DNA comprising the DNA encoding the genome of the virus. The present invention relates to use of DNA encoding the genome of the vector to produce the Paramyxovirus vector of the invention. Reconstitution of a virus from virus vector DNA can be performed according to the known methods (WO97/16539 WO97/16538; Durbin A. P. et al., Virol., 1997, 235, 323-332; Whelan S. P. et al., Proc. Natl. Acad. Sci. USA, 1995, 92, 8388-8392; Schnell M. J. et al., EMBO J., 1994, 13, 4195-4203; Radecke F. et al., EMBO J., 1995, 14, 5773-5784; Lawson N. D. et al., Proc. Natl. Acad. Sci. USA, 1995, 92, 4477-4481; Garcin D. et al., EMBO J., 1995, 14, 6087-6094; Kato A. et al., Genes Cells, 1996, 1, 569-579; Baron M. D. and Barrett T., J. Virol., 1997, 71, 1265-1271; Bridgen A. and Elliott R. M., Proc. Natl. Acad. Sci. USA, 1996, 93, 15400-15404). These methods enable the reconstitution of Paramyxovirus vectors including the parainfluenza virus, vesicular stomatitis virus, rabies virus, measles virus, rinderpest virus, and Sendai virus vectors from DNA. If the F, HN, and/or M genes are deleted from the virus vector DNA, infective virus particles will not be formed. However, it is possible to generate infective virus particles by introducing these deleted genes and/or genes encoding an envelope protein from another virus into the host cells and expressing them.

Methods for introducing vector DNA into cells may include (1) forming DNA precipitates that can be incorporated into desired cells, (2) making a positively charged complex comprising DNA, a complex that is suitable for incorporation by the desired cells and that has low cytotoxicity, and (3) instantaneously opening a pore large enough for DNA to pass through in the desired plasma membrane using an electrical pulse.

A variety of transfection reagents can be used in (2) , for instance, DOTMA (Boehringer), Superfect (QIAGEN #301305), DOTAP, DOPE, and DOSPER (Boehringer #1811169). For (1), transfection using calcium phosphate can be used. In this method, DNA incorporated by cells is taken up into phagocytic vesicles, but it is known that a sufficient amount of DNA is also taken up into the nucleus (Graham F. L. and van Der Eb J., Virol., 1973, 52, 456; Wigler M. and Silverstein S., Cell, 1977, 11, 223). Chen and Okayama studied the optimization of the transfer technology and reported (1) that maximal efficiency is obtained when cells and precipitates are incubated under 2 to 4% CO₂ at 35°C for 15 to 24 hr, (2) that circular DNA has higher activity than linear DNA, and (3) that the optimal precipitates are formed when the DNA concentration in the mixed solution is 20 to 30 µg/ml (Chen C. and Okayama H., Mol. Cell. Biol., 1987, 7, 2745). The method of (2) is suitable for transient transfection. More classically, a transfection method in which DEAE-dextran (Sigma #D-9885 M. W. 5 x 10⁵) is mixed with DNA at a desired concentration ratio is known. Because most complexes are degraded in the endosome, chloroquine may be added to enhance the transfection efficiency (Calos M. P., Proc. Natl. Acad. Sci. USA, 1983, 80, 3015). The method of (3), called electroporation, may be more broadly applied than the methods of (1) and (2) because it can be used for any kind of cells. The transfection efficiency can be maximized by optimizing the duration of pulse currents , the form of pulse, the strength of the electrical field (gap between electrodes, and voltage) , conductivity of buffer, DNA concentration, and cell density.

Among the above three methods, the method of (2) is suitable for the present invention because it is easy to perform and enables the testing of a large number of samples using a large amount of cells. Preferably, transfection reagents such as the Superfect Transfection Reagent (QIAGEN, #301305) or the DOSPER Liposomal Transfection Reagent (Boehringer Mannheim #1811169) are used.

Specific procedures of the reconstitution from cDNA are as follows.

LLC-MK2, a cell line derived from the monkey kidney, is cultured in a 24-well to 6-well plastic plate or in a 100-mm petri dish in minimum essential medium (MEM) containing 10% fetal calf serum (FCS) and an antibiotic (100 units/ml penicillin G and 100 µg/ml streptomycin) to be 70 to 80% confluent. Cells are then infected, for instance, at 2 pfu/cell with recombinant vaccinia virus vTF7-3 that expresses T7 polymerase, which has been inactivated by a 20-minute UV exposure in the presence of 1 µg/ml psoralen (Fuerst T. R. et al., Proc. Natl. Acad. Sci. USA, 1986, 83, 8122-8126; Kato. A. et al., Genes Cells, 1996, 1, 569-579). The amount of psoralen and the duration of UV exposure can be optimized. One hour after infection, cells are transfected by, for example, lipofection using Superfect (QIAGEN) with 2 to 60 µg of, or more preferably 3 to 5 µg of the above recombinant Sendai virus cDNA together with expression plasmids for virus proteins (24-0.5 µg pGEM-N, 12-0.25 µg pGEM-P, and 24-0.5 µg pGEM-L, or more preferably 1 µg pGEM-N, 0.5 µg pGEM-P, and 1 µg pGEM-L) (Kato. A. et al., Genes Cells, 1996, 1, 569-579) that function in trans and are required for producing a full length Sendai virus genome. The transfected cells are cultured in serum free MEM containing, if desired, 100 µg/ml rifampicin (Sigma) and cytosine arabinoside (AraC) (Sigma) whose concentration is more preferably 40 µg/ml, so that the drug concentration is adjusted to be optimal to minimize the cytotoxicity of the vaccinia virus and maximize the recovery of virus (Kato. A. et al., Genes Cells, 1996, 1, 569-579). Cells are cultured for 48 to 72 hr after transfection, then collected and lysed through three cycles of freeze-thawing. The cell lysates are transfected into LLC-MK2 cells, and after a 3- to 7-day culture, the culture medium is collected. To reconstitute a virus vector lacking a gene encoding an envelope protein that is incapable of replication, the vector may be transfected into LLC-MK2 cells expressing an envelope protein, or co-transfected with expression plasmid for the envelope protein. Alternatively, transfected cells can be overlaid and cultured on LLC-MK2 cells expressing envelope protein to propagate a deletion virus vector (PCT/JP00/03194 and PCT/JP00/03195). Alternatively, the above cell lysates prepared by freeze-thawing can be innoculated into the chorioallantoic membrane of an embryonated chicken egg of ten days to collect the allantoic solution after about three days. The virus titer of the culture medium or the allantoic solution can be determined by measuring hemagglutinin activity (HA). The HA may be determined by "endo-point dilution" (Kato. A. et al., Genes Cells, 1996, 1, 569-579; Yonemitsu Y. and Kaneda Y., Hemagglutinating virus of Japan-liposome-mediated gene delivery to vascular cells., Molecular Biology of Vascular Diseases. Methods in Molecular Medicine, Ed. by Baker A. H., Humana Press, 1999, 295-306). To eliminate the possible contamination of vaccinia virus vTF7-3, the obtained allantoic sample may be diluted appropriately (10⁶ times for instance) and re-amplified in chicken eggs. Re-amplification may be repeated, for example, three times or more. The obtained virus stock can be stored at -80°C. The titer of recovered Sendai virus is usually between 10⁸ to 10⁹ pfu/ml, and that of contaminating vaccinia virus vTF7-3 is usually not more than 10³ to 10⁴ pfu/ml.

Host cells are not limited to any special types of cells as long as the virus vector can be reconstituted in the cells. Host cells may include LLC-MK2 cells, CV-1 cells derived from the monkey kidney, cultured cell lines such as BHK cells derived from hamster kidney, and human-derived cells. To obtain a large quantity of the Sendai virus vector, embryonated chicken eggs may be infected with virus vectors obtained from the above host cells and the vectors can be amplified. The method of producing virus vectors using chicken eggs has been established (Advanced protocols in neuroscience study III, Molecular physiology in neuroscience., Ed. by Nakanishi et al., Kouseisha, Osaka, 1993, 153-172). Specifically, for example, fertilized eggs are incubated for 9 to 12 days at 37 to 38°C in an incubator to grow the embryos. Virus vectors are inoculated into the chorioallantoic cavity, and eggs are further incubated for several days to propagate the vectors. Conditions such as the duration of incubation may vary depending on the type of recombinant Sendai virus used. Then, the allantoic solution containing viruses is recovered. Sendai virus vector is separated and purified from the allantoic sample according to the standard method (Tashiro M., Protocols in virus experiments., Ed. by Nagai and Ishihama, MEDICAL VIEW, 1995, 68-73).

For instance, a Sendai virus vector lacking the F protein can be constructed and prepared as follows (PCT/JP00/03194 and PCT/JP00/03195).

### (1) Construction of Sendai virus genome cDNA lacking the F gene and an expression plasmid for F gene

Full length Sendai virus (SeV) genomic cDNA, pSeV18⁺b(+) (Hasan M. K. et al., J. General Virol., 1997, 78, 2813-2820) (pSeV18⁺b(+) may be also called pSeV18⁺) , is digested with SphI and KpnI, and the resulting fragment (14673 bp) is recovered and cloned into pUC18 to obtain pUC18/KS. pUC18/KS is used for constructing a region lacking the F gene. Deletion of the F gene is performed by combination of PCR-ligation, and the ORF of the F gene (1698 bp, from ATG to TGA) is replaced with the sequence 5'-atgcatgccggcagatga (SEQ ID NO: 4) in the resulting F gene-deleted SeV genomic cDNA (pSeV18⁺/ΔF). PCR products obtained using primers (forward: 5'-gttgagtactgcaagagc/SEQ ID NO: 5; reverse: 5'-tttgccggcatgcatgtttcccaaggggagagttttgcaacc/SEQ ID NO: 6) and those with primers (forward: 5'-atgcatgccggcagatga/SEQ ID NO: 7; reverse: 5'-tgggtgaatgagagaatcagc/SEQ ID NO: 8) are digested with EcoT22I and cloned into the upstream and downstream of the F gene, respectively. The resulting plasmid is digested with SacI and Sall, and the fragment containing the F gene deletion site (4931 bp) is recovered and cloned into pUC18 to obtain pUC18/dFSS. pUC18/dFSS is digested with DraIII, and the fragment recovered is replaced with the DraIII fragment of pSeV18⁺ that contains F gene, and ligated to obtain pSeV18⁺/ΔF.

A foreign gene can be inserted into the NsiI or NgoMIV site in the F gene deletion site of pUC18/dFSS. For this purpose, a fragment containing a foreign gene may be amplified using NsiI-tailed primers or NgoMIV-tailed primers.

### (2) Preparation of helper cells for inducible expression of SeV-F protein

A Cre/loxP inducible expression plasmid for the Sendai virus F gene (SeV-F) is constructed as follows. SeV-F gene is amplified by PCR, and cloned into the unique SwaI site of the pCALNdLw plasmid (Arai et al., J. Virol., 1998, 72, 1115-1121), which is designed for inducible expression of gene products through the function of Cre DNA recombinase, to obtain pCALNdLw/F.

To recover infective virus particles from the F gene-deleted genome, a helper cell line expressing SeV-F protein is established. LLC-MK2 cells, derived from the Simian kidney and commonly used for SeV propagation, may be used. LLC-MK2 cells are cultured at 37°C, 5% CO₂ in MEM containing 10% heat-inactivated and immobilized fetal bovine serum (FBS), 50 U/ml of penicillin G sodium, and 50 µg/ml streptomycin. Because of the cytotoxicity of the SeV-F gene product, the gene is cloned into the pCALNdLw, where the expression of a cloned gene is inducible by Cre DNA recombinase. The above pCALNdLw/F is used for transfecting LLC-MK2 cells by the calcium phosphate method (mammalian transfection kit (Stratagene)) according to the standard protocol.

LLC-MK2 cells grown in 10-cm plates to be 40% confluent are transfected with 10 µg pCALNdLw/F and incubated in 10 ml of MEM containing 10 % FBS at 37°C under 5% CO₂ for 24 hr. Then, cells are dispersed, resuspended in 10 ml of culture medium, and plated onto five 10-cm dishes, where 5 ml of cell suspension is plated onto one dish, 2 ml onto two, and 0.2 ml onto two. Cells are cultured in 10 ml of MEM containing 10% FBS plus 1200 µg/ml G418 (GIBCO-BRL) for 14 days with medium changed every two days, and stable transfectants are selected. Cells grown in the medium that are resistant to G418 are recovered using cloning rings. Cells of each clone are further cultured until they grow to be 100% confluent in a 10-cm dish.

To induce F protein expression, cells are grown to be 100% confluent in 6 cm dishes, and infected with AxCANCre adenovirus at moi = 3 according to the method by Saito et al. (Saito et al., Nucleic Acids Res., 1995, 23, 3816-3821; Arai T. et al., J. Virol., 1998, 72, 1115-1121).

### (3) Reconstitution and propagation of the F gene-deleted SeV virus

The pSeV18⁺/ΔF into which a foreign gene has been inserted is transfected into LLC-MK2 cells as follows. Cells are plated at 5 x 10⁶ cells/dish onto 100-mm petri dishes, cultured for 24 hr, and then infected at room temperature for 1 hr with the recombinant vaccinia virus that expresses T7 RNA polymerase and that has been treated with psoralen and long UV (365 nm) for 20 min (Fuerst T. R. et al., Proc. Natl. Acad. Sci. USA, 1986, 83, 8122-8126) (moi = 2) (moi = 2 to 3; preferably moi = 2). UV exposure may be performed using UV Stratakinker 2400 equipped with five 15-watt bulbs (catalogue number 400676 (100 V) , Stratagene, La Jolla, CA, USA) . After cells are washed three times, plasmids pSeV18⁺/ΔF-GFP, pGEM/NP, pGEM/P, and pGEM/L (Kato A. et al., Genes Cells, 1996, 1, 569-579) are resuspended with OptiMEM (GIBCO) at a ratio of 12 µg/dish, 4 µg/dish, 2 µg/dish, and 4 µg/dish, respectively and mixed with SuperFect transfection reagent (5 µl SuperFect (QIAGEN) for 1 µg DNA). The mixture is incubated for 10 min at room temperature, then resuspended with 3 ml of OptiMEM with a final concentration of 3% FBS, and added to the cells. After a 3-hr culture in an incubator, cells are washed twice with serum free MEM, and further cultured in MEM containing 40 µg/ml of cytosine β-D-arabinofuranoside (AraC, Sigma) and 7.5 µg/ml of trypsin (GIBCO) for 70 hr. Then, cells are collected and resuspended in OptiMEM at 10⁷ cells/ml. Cells are frozen-thawed three times, then mixed with lipofection reagent DOSPER (Boehringer mannheim) (10⁶ cells per 25 µl DOSPER), incubated at room temperature for 15 min, and transfected into LLC-MK2/F7 cells (10⁶ cells/well in 12-well-plate), which is one of the clones of F gene-expressing helper cells selected as described above. Cells are cultured in serum free MEM containing 40 µg/ml of AraC and 7.5 µg/ml of trypsin, and the culture supernatant is collected.

In preparing deletion virus vectors, two different virus vectors having deletion of a different envelope gene may be transfected into the same cell. In this case, each deleted envelope protein is supplied through expression from the other complex, and this mutual complementation permits the generation of infective virus particles, which can replicate and propagate. Thus, two or more of the virus vectors of the present invention may be simultaneously inoculated in a combination that complement each other, thereby producing a mixture of each envelope deletion virus vector at a low cost and in a large scale. Because these viruses lacking an envelope gene have a smaller genome, they can allow the insertion of a long foreign gene. In addition, it is difficult for these viruses, which are intrinsically non-infective, to keep the status of co-infection after being diluted outside cells, and thus they are sterilized and less harmful to the environment.

If a vector is prepared using as the foreign gene a therapeutic gene for a specific disease, gene therapy can be done by administering this vector. The virus vector of the present invention enables the expression of a foreign gene that can help cure a disease, an endogenous gene that is lacking or insufficient in patients, and such.

The virus vector of the present invention can be made as a composition together with a desired, pharmaceutically acceptable carrier. Herein, a "pharmaceutically acceptable carrier" is defined as those materials that can be administered with a vector, but does not inhibit gene transfer by the vector. For instance, the virus vector may be appropriately diluted with saline, phosphate buffered saline (PBS), and so on to make a composition . If the virus vector is propagated in chicken eggs, the composition may contain an allantoic solution. The allontoic solution can be appropreately diluted with saline, phosphate buffered saline (PBS), etc. Also, the composition may contain carriers such as deionized water or a 5% dextrose aqueous solution. It may further contain stabilizers, antibiotics, or the like. The composition of the present invention comprises a pharmaceutical composition. Thus, the present invention also relates to the use of the virus vector or the above composition as a pharmaceutical.

The above-obtained recombinant Paramyxovirus vector, or a composition comprising said vector contacts with blood vessels to transfect a foreign gene harbored within the vector into vascular cells. Infectious Paramyxovirus vector is capable of transferring nucleic acid into cells with short exposure of the vector to blood vessels. Although there is no limitation on the vascular cells that are targeted by the gene transfer using Paramyxovirus vector, preferable examples of them include endothelial cells in the lumen, endothelial cells in vasa vasora (vessel wall nutrient vessel), vascular smooth muscle cells in vascular media, and/or adventitial cells.

Specifically, the vector of the invention may be used for therapies targeted at the vascular cells in the following disorders and injures:
(1) restenosis after PTCA,
(2) late occlusion after bypass surgery using autografted vein,
(3) vasospasm,
(4) transfer of genes encoding such as thrombolytic protein, or anticoagulant protein (e.g. tissue plasminogen activator, tissue factor pathway inhibitor (TFPI) , etc.) for the treatment of thrombosis,
(5) transfer of genes encoding angiogenic factors for the treatment of ischemic disease (e.g. ischemic arteriosclerosis, stenocardia, etc.).

Foreign genes that are particularly useful in gene transfer targeted at vascular cells (e.g. in gene therapies) may include those encoding:
(1) cell cycle inhibitor (e.g. p53, p21, p16 and p27),
(2) inhibitor of cell proliferation signal (e.g. mutant H-Ras),
(3) secretory cell proliferation inhibitor (e.g. eNOS and CNP (C type sodium diuretic peptide)),
(4) vascular smooth muscle relaxing factor (e.g. eNOS and CNP),
(5) vascular smooth muscle relaxing ion channel (e.g. C-terminal deletion mutant of Kir 6.2 potassium ion channel),
(6) thrombolytic protein (e.g. tissue plasminogen activator and urokinase),
(7) tissue factor pathway inhibitor (e.g. TFPI), and
(8) angiogenic factor (e.g. VEGF, FGF, and HGF).

For gene therapy, a composition containing the Paramyxovirus vector may be administered *in vivo* from the interior or exterior of the lumen into diseased vessels , and foreign genes are to be expressed in vascular cells. Alternatively, administration may be performed *ex vivo*. *In vivo* gene transfer may be performed by, for example, simple floating method into the vascular lumen, injection into the luminal space with positive pressure, or local administration such as drops into blood vessels. Alternatively, gene transfer may be performed by transferring a gene into the vascular lumen while blocking blood flow using the double balloon catheter, injecting forcefully using the infusion balloon catheter into the vascular smooth muscle layer, or pressing a vector to the vascular lumen using the hydrogel balloon catheter.

The vector of the present invention can efficiently transfer genes into the medial layers when administered with protease. Preferable examples of proteases to be used include those which digest the extracellular matrix of the blood vessel (such as those in the endothelium and media). Such protease may include matrix metalloproteases (MMPs), which include approximately 20 members identified in humans (Seiki M. APMIS 107:137-143 (1999)), 4 types of amphibian collagenase (Stolow M.A. et al., Mol. Biol. Cell 7:1471-1483 (1996)), sea urchin hatching enzyme (Nomura K. et al., Biochemistry 36 (23) : 7225-7238 (1997)), and *Clostridium histolyticum* collagenase (Yoshida E. and Noda H. Biochim. Biophys. Acta 105(3) : 562-574 (1965)); elastase; plasmin; tissue plasminogen activator (tPA) ; urokinase; cathepsin (B, L, D, and G) ; trypsin; and thrombin, but is not limited thereto. Among them, preferred proteases to be used in administration of the vector of the present invention are collagenase, urokinase, tPA, elastase, plasmin, and MMPs. More preferably, collagenase, plasmin, and MMPs may be used. Collagenase may be type 4 collagenase (e.g. *Clostridium histolyticum* collagenase), and MMPs may be MMP2 or MMP9. MMPs may be activated by treatment with aminophenylmercuric acetate (APMA). MMP2 or plasmin may be preferred for transferring vector from the interior of the blood vessel having enlarged intimal layers containing fibrin under basal lamina. A protease may be used alone or in combination with other proteases.

Nucleic acid transfer into the vascular cells using protease and the vector of the invention may be performed by a method comprising
(a) treating tissues containing vascular cells with a protease, and
(b) contacting a recombinant Paramyxovirus vector or cells containing such vector with the vascular cells, in which the steps (a) and (b) are carried out sequentially or simultaneously.

Treating with protease before administration of the vector into vascular cells may be preferred to achieve high efficiency of transfection. However, in clinical application where the time for blocking blood flow is limited, protease and vector may be mixed and administered simultaneously. In the present invention, it was confirmed that the vector infectivity into the medial layer is significantly enhanced by simultaneous administration, which simplifies the procedures for administration. Time for protease treatment may be determined so as to increase the vector infectivity, and is not limited. For instance, protease may be applied for a desired time between about 5 to 20 min at the indicated concentrations specified in the following Examples. Since high concentrations of collagenase may potentially cause physiological thrombosis, collagenase may be preferably used at a concentration not more than 5000 unit/ml, for instance, at 500 unit/ml. However, the concentration of protease and the time for treatment can be selected appropriately. Protease treatment may be performed at 37°C. Time for infection using vector after protease treatment may be chosen so as to ensure that the vector can be transfected into the target vascular cells, and is not limited. In clinical applications, it is desired to minimize the time for infection so as to shorten the time in which blood flow is blocked. Time for infection may be several minutes, for instance, and specifically, a desired time between about 1 to about 10 min. The vector of the invention is capable of obtaining significant infection with 1 min exposure or longer. For instance, in gene transfer into the coronary artery, the time for blocking blood flow is desirably not more than 5 min, and, for instance, short period of time between about 1 to 5 min may be effectively used. *In vivo* gene transfer into the vascular lumen using protease treatment and vector administration may be performed by transferring a gene into the vascular lumen while blocking blood flow using the double balloon catheter, injecting forcefully using the infusion balloon catheter into the vascular smooth muscle layer, or pressing a vector to the vascular lumen using the hydrogel balloon catheter as described above. The infectivity may be improved by ablating the vascular endothelium before administration.

The transfection efficiency of virus vector may be further increased by administrating virus vector together with biocompatible polyol (such as poloxamer 407) (March K.L. et al., Human Gene Ther. 6:41-53 (1995)). Thereby, the dose of virus vector can be reduced, or the time for infection may be shortened. In the present invention, biocompatible polyol may be combined with the Paramyxovirus vector of the invention to make a composition. Other type of biocompatible polyol may also be used. These agents may be administered with vector simultaneously or separately.

The vector of the present invention may be effectively administered in a form of matrix. In a known method, virus vector is suspended in atelocollagen matrix, and solidified to mini pellets by freeze-drying. This method utilizes the gradual degradation of the matrix and is reported to enable persisting the effect of adenovirus vector and naked DNA, whose expression is known to be transient (Ochiya T. et al., Nature Medicine 5:707-710 (1999)). The virus vector of the present invention can be mixed with such auxiliaries, and stored by freeze-drying. The vector may also be mixed with cationic lipid to enhance the level of expression.

It is known that a matrix so small as to be administered itself is capable of releasing such as growth factor gradually through the 18G needle over a long period. For instance, preparation comprising such proteins as growth hormone has substantially longer duration in blood vessels than those administered alone. For instance, it may be 7 days or longer, but normally can be 10 days or longer (Unexamined Published Japanese Patent Application No. Hei 10-001440). Accordingly, the numbers of administration and pain to the patients are markedly reduced. The preparation described above may be used as, for example, solid injection for subcutaneous and intramuscular administration (such as implants), or as mucoabsorbent such as suppository. The dosage form for injection may be column or granule that can be administered through a needle. Columnar form such as rectangular or cylindrical column, or spherical form may be used preferably.

The size of a parenteral preparation of the present invention may be selected depending on the administration method, but not limited as long as it does not bring excessive pain to patients. Injections composed of columnar matrix may be, for example, not more than 3 mm in diameter (e.g. 0.1 to 3 mm), and not more than 30 mm in length (e.g. 0.5 to 30 mm), preferably as small as not more than 1.3 mm in diameter (e.g. 0.1 to 1.2 mm) and not more than 20 mm in length (e.g. 0.5 to 20 mm), which facilities injection through a 14G-needle or smaller, and more preferably a cylindrical column of 0.1 to 1 mm in diameter and 1 to 20 mm in length. Injections composed of spherical matrix may be not more than 1 mm (e.g. 0.1 µm to 1 mm), preferably not more than 150 µm (e.g. 0.5 to 100 µm), and more preferably between 1 to 100 µm, in the maximal diameter. The weight of matrix can be selected according to the form of preparation, for instance, not more than 40 mg, and preferably between 1 to 25 mg for injections.

The virus vector of the invention may be administered at a sufficient dose so that an effective dose of vectors can be transferred to vascular cells. Herein, the "effective dose" is defined as a dose that enables the introduction of genes to the cells of the target tissue so as to bring, at least partially, the desired therapeutic effect or preventive effect of the present invention. The administration of an effective dose of the virus vector containing a desired gene enables the transfected cells to produce the gene product. Dose of the vector used for administration may vary depending on the disease, the body weight, age, sex, symptom, the purpose of administration, and the transfected gene, and so on, but it can be appropriately determined by those skilled in the art. The dose of the vector may be preferably within the range of approximately 10⁵ pfu/ml to 10¹¹ pfu/ml, and more preferably approximately 10⁷ pfu/ml to 10⁹ pfu/ml, but most preferably, the vector is administered at approximately 1 x 10⁸ pfu/ml to 5 x 10⁸ pfu/ml with pharmaceutically acceptable carriers.

The composition of the present invention containing the virus vector may be administered into subjects such as all mammalian animals including humans, monkeys, mice, rats, rabbits, sheep, cattle, and dogs.

### Brief Description of the Drawings

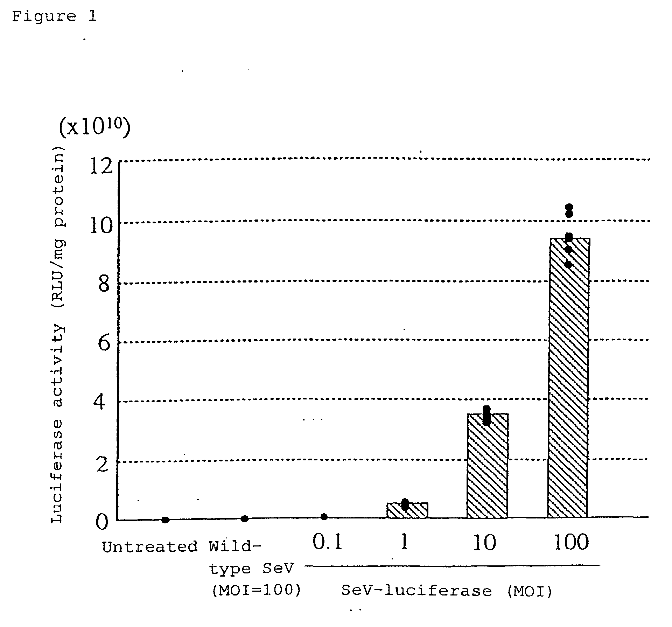

Figure 1 is a bar graph showing the dose-dependent transfection efficiency of SeV-luc into BSMC. Cells were seeded in 6-well plates at 10⁵ cells/well, and SeV-luc at MOI=0.1, 1, 10, or 100, or wild-type SeV at MOI=100 (n=6 for each) was added. After 48 hr, cells were subjected to the luciferase assay. Data are expressed as means ± S.D. Each dot represents the value from each well normalized by the protein concentration.

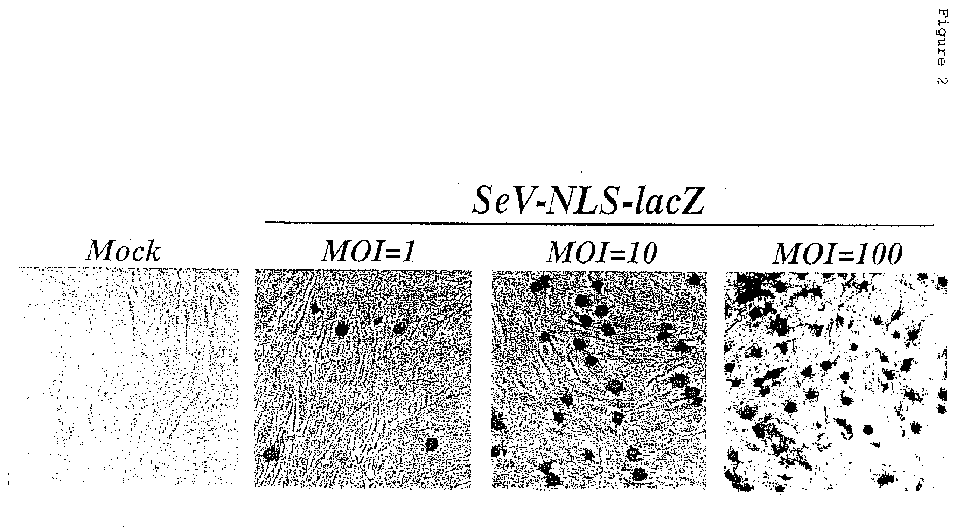

Figure 2 shows phase contrast microscopy photographs of BSMC that had been exposed to wild-type SeV (MOI=100) or various doses of SeV-NLS-lacZ (MOI=1, 10, and 100). The photographs show the dose-dependent transfection efficiency (n=6 for each). Forty-eight hours after gene transfer, cells were fixed in 2% paraformaldehyde plus 0.25% glutaraldehyde for 10 min, and incubated in X-Gal solution at room temperature for 1 hr. The number of cells containing nuclei clearly stained blue increased in dose-dependent manner. Original magnification: 100x.

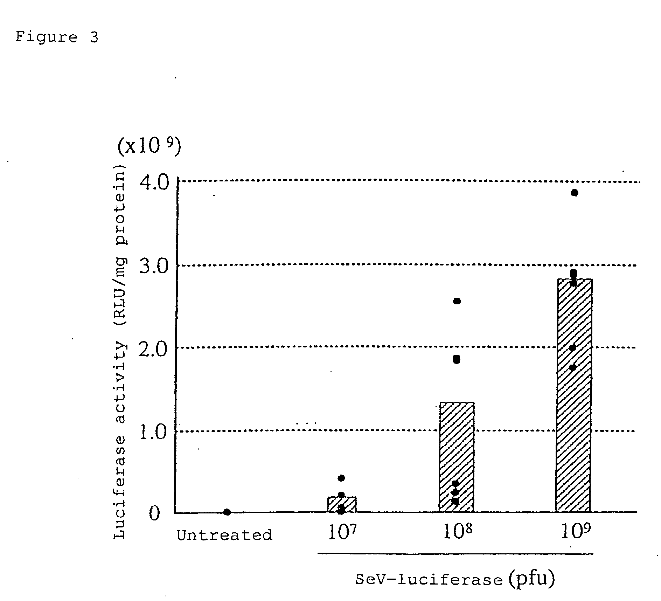

Figure 3 is a bar graph showing the dose-dependent transfection efficiency of SeV-luc into human saphenous vein. Human saphenous vein, 5 to 10 mm in length, was washed extensively and immersed in vector solution containing SeV-luc at 10⁷, 10⁸, or 10⁹ pfu (plaque forming unit) /ml, or vector solution without virus . After 48 hr, the infected vein was subjected to the luciferase assay. Data are expressed as means ± S.D. Each dot represents the value from each well normalized by the protein concentration.

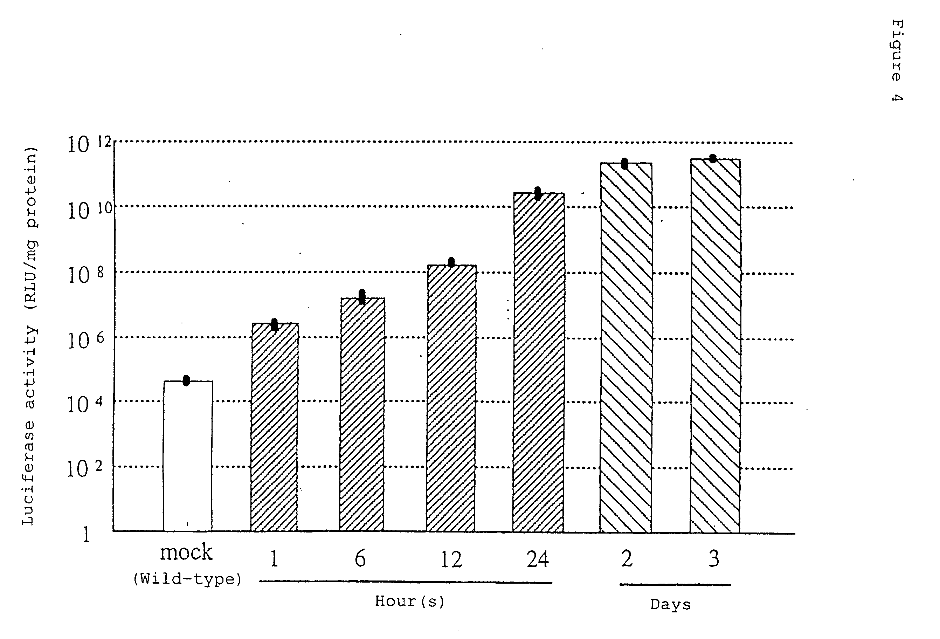

Figure 4 is a bar graph showing the time-dependent increase in reporter gene expression in BSMC after transfection with SeV-luc or wild-type SeV (at MOI=10) (n=6 for each). Cells were harvested at the indicated time points after transfection, and subjected to the luciferase assay. The expression level of the transfected gene reached a plateau 2 or more days after transfection. Data are expressed as means ± S.D and shown in logarithmic scale. Each dot represents the value from each well normalized by the protein concentration.

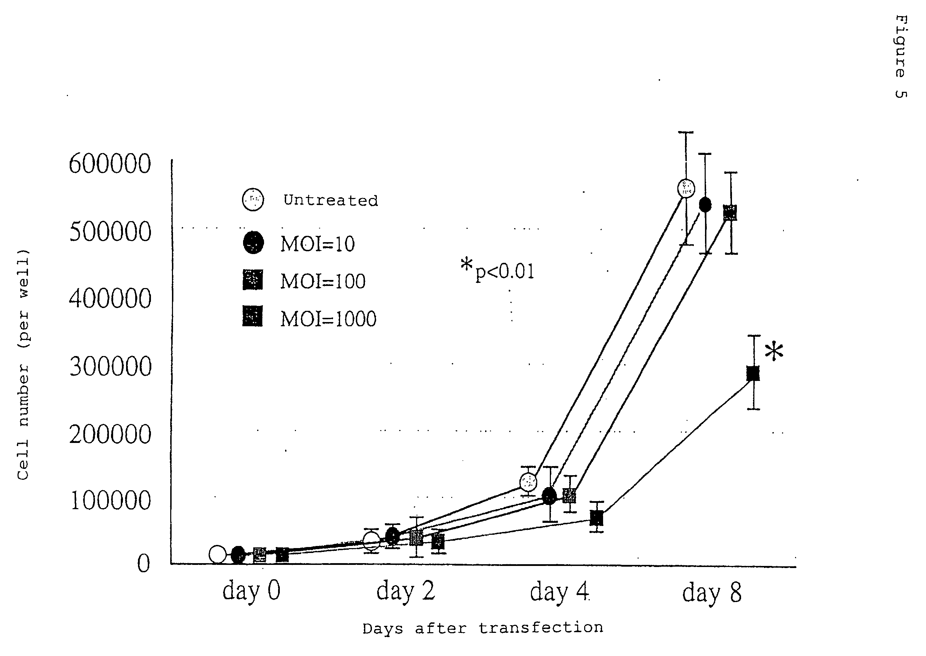

Figure 5 shows the effect of SeV infection on the proliferation activity of BSMC. After plated, cells were infected with various doses of recombinant wild-type SeV. The cell number was counted at the indicated time points. At MOI=1000, proliferation was significantly inhibited.

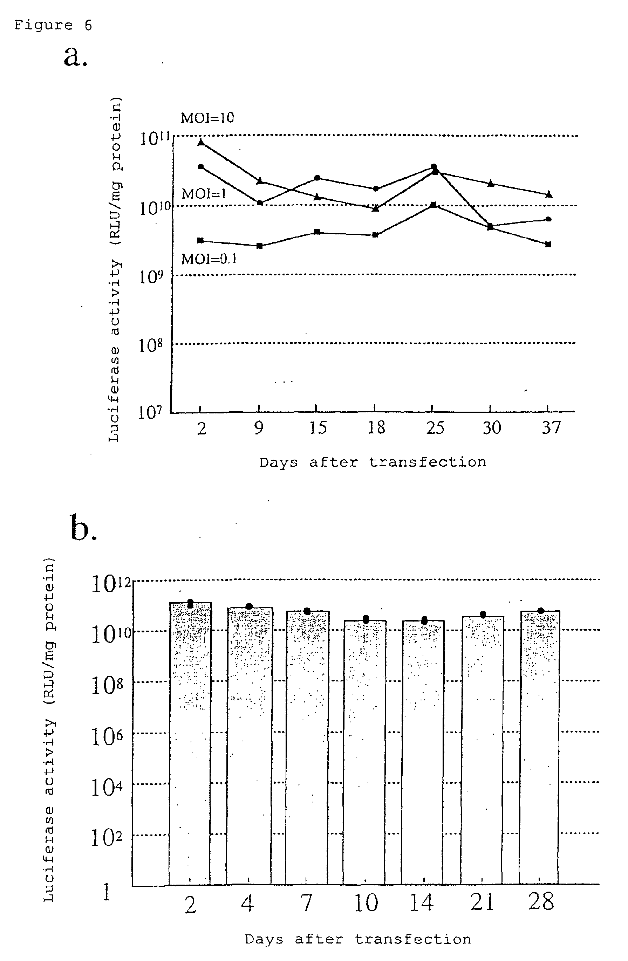

Figure 6 shows the time course of the expression of a foreign gene.
   (a) shows the genomic stability in BSMC during the logarithmic phase. Cells were exposed to the various concentrations of SeV-luc (MOI=0.1, 1, and 10), and immediately before they reach 100% confluency, three quarters of the cells were subjected to the luciferase assay. The rest of the cells were subjected to passage culture. Each dot represents the value at the indicated time point, which was normalized by the protein concentration. The experiments were performed in triplicate, and the representative results were shown. Data are expressed in logarithmic scale.
   (b) shows the genomic stability in quiescent BSMC. The culture of BSMC was rendered to be confluent, and left for additional two days. Cells were exposed to SeV-luc (MOI=10) for lhr and subjected to the luciferase assay at the indicated time points (n=6 for each). Data are expressed as means ± S.D and shown in logarithmic scale. Each dot represents the value from each well normalized by the protein concentration.

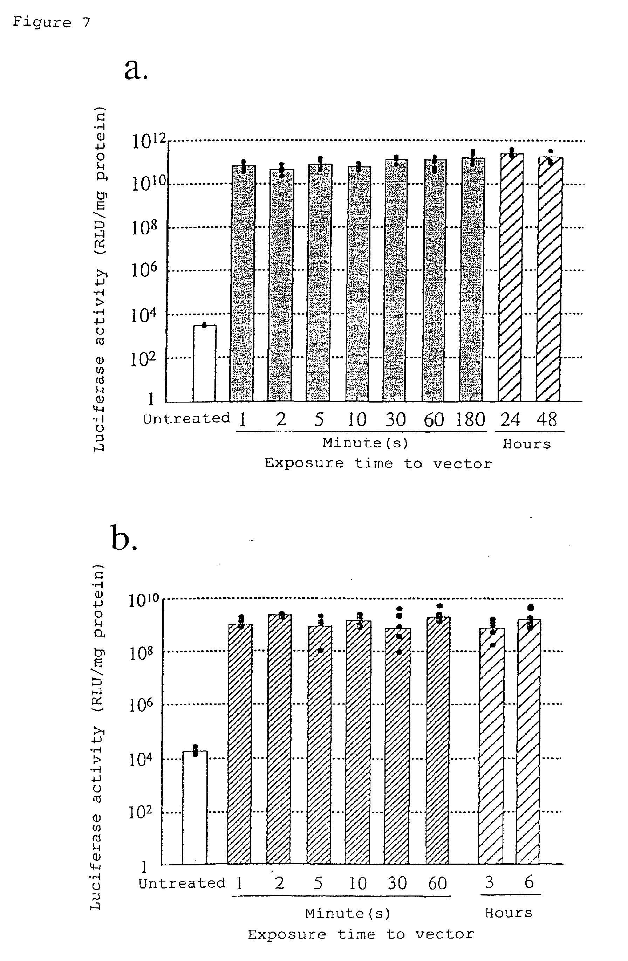

Figure 7 is a graph showing the effect of vector exposure time on SeV-mediated gene transfer.
   (a) is a graph showing the effect of vector exposure time on SeV-mediated gene transfer in BSMC. BSMCs were exposed to SeV-luc (MOI=10) for a different time: 1, 2, 5, 10, 30, 60, or 180 min, or 24 or 48 hr and washed twice by immersing in fresh medium (n=6 for each). After 48 hr, cells were subjected to the luciferase assay. Data are expressed as means ± S.D and shown in logarithmic scale. Each dot represents the value from each well normalized by the protein concentration.
   (b) is a graph showing the effect of vector exposure time on SeV-mediated gene transfer in human saphenous vein. Human great saphenous vein, 5 to 10 mm in length, was exposed to SeV-luc (MOI=10) for 1, 2, 5, 10, 30, or 60 min, or 3 or 6 hr, washed four times by immersing in fresh medium, and then plated in culture plates (n=6 for each) . After 48 hr, the infected vein was subjected to the luciferase assay. Data are expressed as means ± S.D, and shown in logarithmic scale. Each dot represents the value from each well normalized by the protein concentration.
Figure 8 is a graph showing the effect of vector exposure time on recombinant adenovirus vector-mediated gene transfer into BSMC. BSMCs were exposed to AdexCA-lacZ (UenoH. et al., Arterioscler. Thromb. Vasc. Biol. 15:2246-2253 (1995)) at MOI=10 for 1, 2, 5, 10, 30, 60, or 180 min or 24 or 48 hr and washed twice by immersing in fresh medium (n=6 for each). After 48 hr, the infected cells were subjected to the luminescent β-galactosidase assay. The expression of the transfected gene clearly increased depending on the vector exposure time. Data are expressed as means ± S.D, and shown in logarithmic scale. Each dot represents the value from each well normalized by the protein concentration.

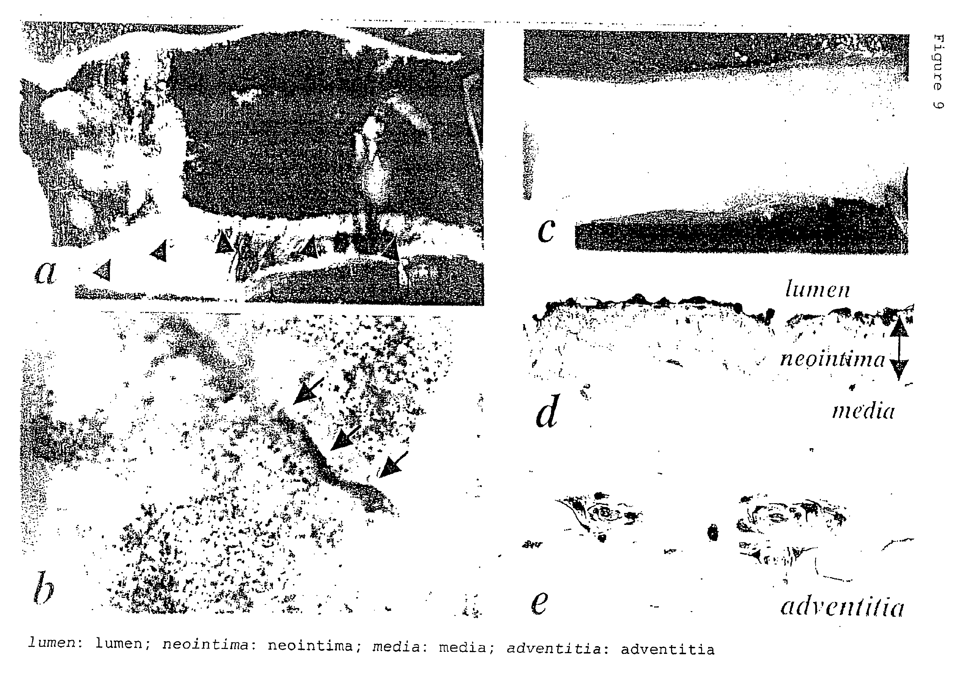

Figure 9 shows stereomicrograph and light micrograph of human saphenous vein expressing β-galactosidase tagged with the nuclear localization signal, which was transfected by exposing SeV-NLS-lacZ (5x 10⁸ pfu) to the lumen of human saphenous vein for 10 min with injection pressure at 150 mmHg (n=6 for each) . After 2 days, the infected vein was fixed in 2% paraformaldehyde plus 0.25% glutaraldehyde for 10 min, and incubated in X-Gal solution at room temperature for 3 hr.
   (a) to (c) show typical stereomicroscopic observations of the vein exposed to SeV-NLS-lacZ with injection pressure at 150 mmHg. Blue spots intensely stained were found at high frequency distributed on the luminal surface (a) and in adventitia (b). The white region looking like a railway indicates the place in which the gene was not transfected, where the endothelium had been removed by injury during operation (a: arrowhead) . A zonal blue line was frequently observed in adventitia, which indicates the gene transfer into vasa vasora (vessel wall nutrient vessel) (b: arrow) . Blue spots were absent from the vein infected with wild-type SeV (c). The original magnifications are 12x for (a), 36x for (b), and 8x for (c).
   (d) and (e) show the histological observations of the same vein as shown in (a) to (c). Most of luminal endothelial cells (d) and some of the adventitial vasa vasora and cells surrounding the vessel (e) were stained blue intensely. Nuclei were counter-stained with fast red. The original magnification is 200x for (d and e).

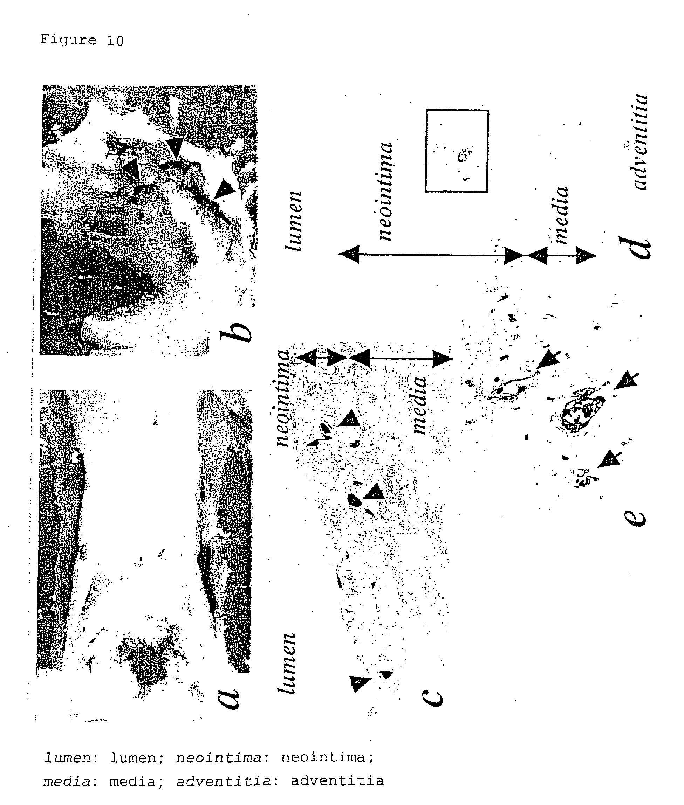

Figure 10 shows photographs for stereomicroscopic observation and light microscopic observation showing the result of examination for β-galactosidase expression in the balloon-injuredvein transfected with SeV-NLS-lacZ (5x 10⁸ pfu) by exposing to the lumen of the balloon-injured vein at injection pressure 150 mmHg for 10 min (n=6 for each). After 2 days, the infected vein was fixed in 2% paraformaldehyde plus 0.25% glutaraldehyde for 10 min and incubated in X-Gal solution for 3 hr at room temperature.
   (a) and (b) show typical stereomicroscopic observations of the balloon-injured vein exposed to SeV-NLS-lacZ at injection pressure 150 mmHg. The blue spots on the luminal surface disappeared markedly, and became sporadic (a, Compare with Figure 9a) . In addition, zonal blue lines were also found in adventitia, indicating the gene transfer into vasa vasora (b: arrowhead) . The original magnifications are 8x for (a) and 12x for (b).
   (c) to (e) show the histological observations of the same vein as shown in (a) and (b). Most of the luminal endothelial cells were ablated, and accordingly, the vein was negative for X-Gal staining, whereas blue cells were found in thin neointima sporadically (c: arrow) .
   (d) shows histological observations of another vein containing thick neointima. Note that blue cells were absent from the luminal surface and neointima except some blue cells in boxed area.
   (e) shows a higher magnification image of (d). Some of the neointimal capillaries were positive for X-Gal staining. Nuclei were counter-stained by fast red. The original magnification is 200x for (c), 60x for (d), and 200x for (e) .

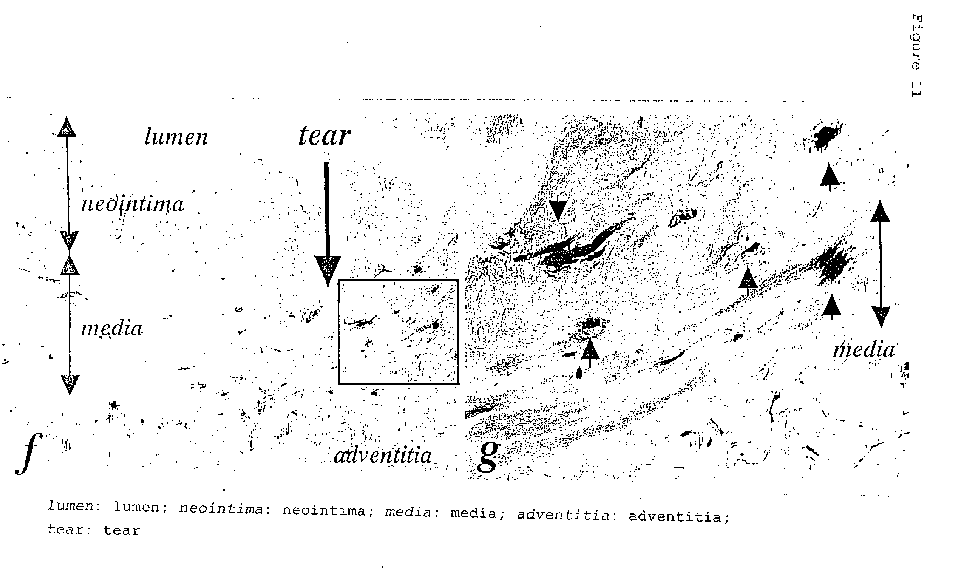

Figure 11 shows photographs for stereomicroscopic observation and light microscopic observation for β-galactosidase expression in the balloon-injured vein, which was transfected by exposing SeV-NLS-lacZ (5x 10⁸ pfu) to the lumen of the vein at injection pressure 150 mmHg for 10 min (n=6 for each) . Two days after transfection, the vein was fixed in 2% paraformaldehyde plus 0.25% glutaraldeyde for 10 min, and incubated for 3 hr in X-Gal solution at room temperature.
   (f) and (g) show histological observations of another vein having neointima torn apart. In (f), as in Figures 10(c) to (e), most of the luminal endothelial cells and the neointimal cells were negative for X-Gal staining, whereas blue cells were present in media and adventitia sporadically. (g) shows a higher magnification image of the boxed area in (f) . Some of the vascular smooth muscle cells with spindle-shape were clearly positive for X-Gal staining (arrow). Nuclei were counter-stained by fast red. The original magnifications are 60x for (f) and 200x for (g).
Figure 12 shows photographs representing the result of *in vivo* gene transfer into rabbit carotid. Two days after SeV-NLS-lacZ (5x 10⁷ pfu) was transfected into the carotid, the carotids were dissected and stained with X-Gal, and then sections were prepared.
Figure 13 shows the result of ex *vivo* SeV vector-mediated gene transfer into rat thoracic artery.
   (a) shows the dose-dependent transfection efficiency of GFP/SeV into rat thoracic artery. Dissected rat thoracic artery was immersed in 100 µl of Hanks solution containing GFP/SeV at the indicated concentrations for 10 min and washed twice. The infected arteries were plated on 24-well plates and cultured in DMEM containing 10% FBS in a CO₂ incubator for 48 hr. The arteries were examined by fluorescence light microscopy, and the cell number per 1 mm² was counted (n=6 for each). Data indicates dose-dependent transfection of GFP/SeV.
   (b) shows time-dependent transfection efficiency of GFP/SeV into rat thoracic artery. Dissected rat thoracic artery was immersed in solution containing GFP/SeV at 1x 10⁸ pfu/ml for the indicated times and washed twice. The arteries were plated on 24-well plates and cultured in DMEM containing 10% FBS in a CO₂ incubator for 48 hr. The arteries were examined by fluorescence light microscopy, and the cell number per 1 mm² was counted (n=6 for each).
   (c) shows a comparison of transfection efficiencies into rat thoracic artery between SeV and adenovirus vectors. Dissected rat thoracic artery was immersed in solution containing LacZ/SeV or LacZ/Adenovirus at the indicate concentrations for 2 min and washed twice. The infected arteries were plated on 24-well plates and cultured in DMEM containing 10% FBS in a CO₂ incubator for 48 hr. The arteries were fixed for 10min and stained for 3 hr using the β-galactosidase staining kit. The cell number per 1 mm² was counted (n=6 for each).

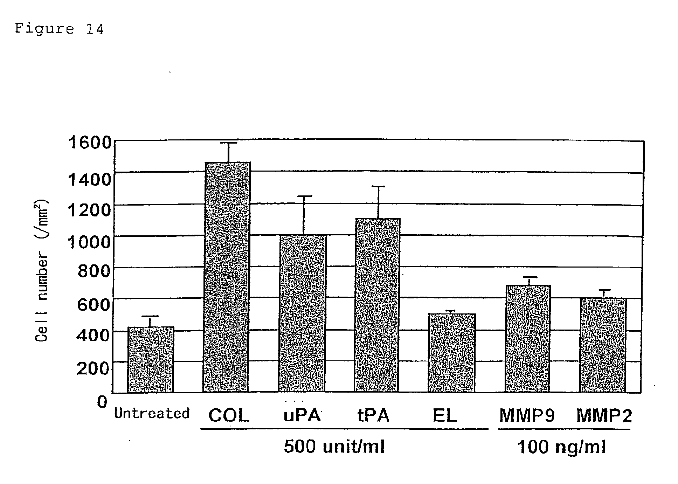

Figure 14 is a diagram representing the elevation of transfection efficiency of SeV into blood vessel by pretreatment with protease. Dissected rat thoracic arteries were pretreated with various proteases for 5 min before transfection. Hanks solution containing protease was pre-warmed for 10 min. The arteries were immersed in Hanks solution containing LacZ/SeV (1x 10⁸ pfu/ml) for 2 min, washed, and cultured for 48 hr. The arteries were stained for 3 hr for the detection of β-galactosidase activity. Collagenase type 4 (COL), urokinase (uPA), tissue plasminogen activator (tPA), elastase (EL) , and matrix metalloproteases (MMP9 and MMP2), which were preactivated with APMA, were used (n=5 for each).

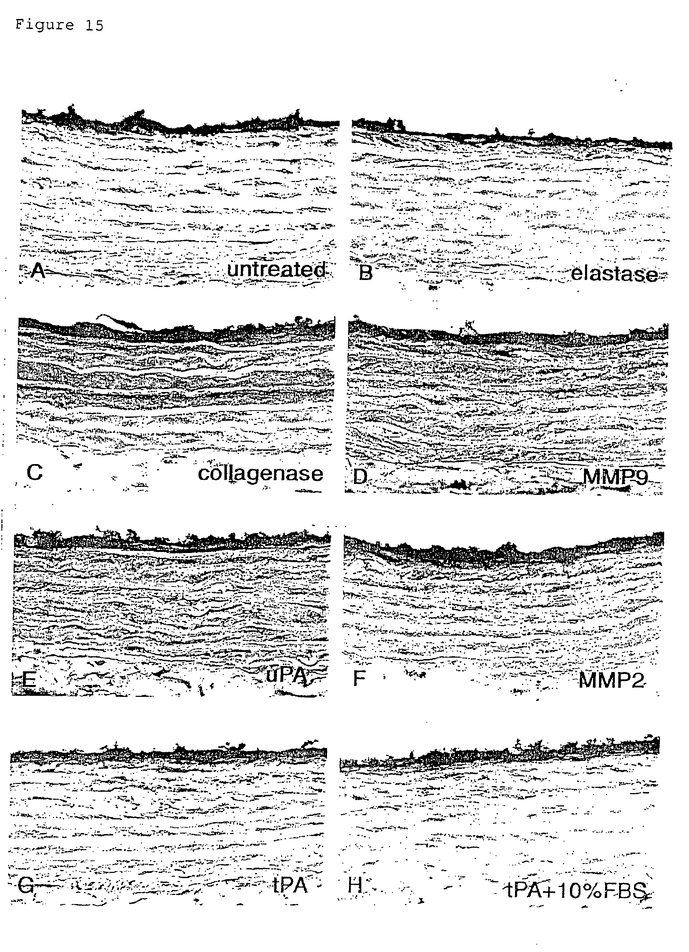

Figure 15 shows photographs representing the result of *ex vivo* SeV vector-mediated gene transfer into rat media by protease pretreatment. Sections of the rat thoracic arteries transfected with SeV in Figure 14 were shown. Nuclei were counter-stained with fast red. The cells successfully transfected were limited to the vascular endothelial cells in the arteries untreated, or those treated with elastase, urokinase, or tPA. In contrast, in the arteries pretreated with collagenase type 4, MMP9, or MMP2, transfected cells were found in the medial vascular smooth muscle cells beyond basal lamina. Enzymes transfected into media are capable of digesting basal lamina, suggesting that digestion of the basal lamina is essential for transfection into media. Moreover, although it was impossible to transfer genes into media using tPA alone, transfer into media was possible in the presence of medium containing 10% FBS. This could be because tPA digested plasminogen in serum, and thereby active plasmin was produced.
   "untreated": without protease treatment, "elastase": treated with elastase, "collagenase": treated with collagenase type 4, "MMP9": treated with activated MMP9, "uPA": treated with urokinase, "MMP2": treated with activated MMP2, "tPA": treated with tPA, and "tPA+10% FBS": treated with tPA in the presence of 10% FBS.

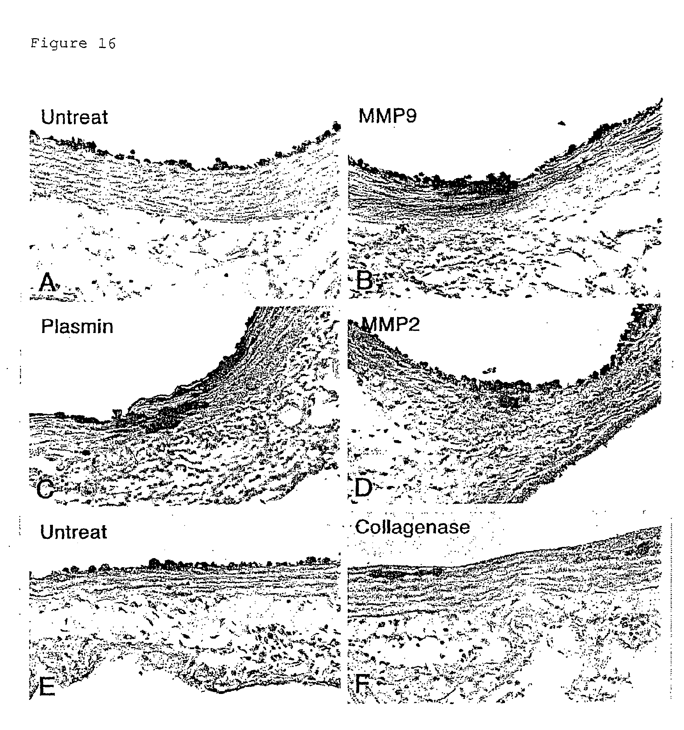

Figure 16 is photographs representing the result of *in vivo* SeV-mediated gene transfer into rat vascular media by protease pretreatment. To examine the effect of protease pretreatment on *in vivo* gene transfer, rat abdominal aorta and carotid arteries were subjected to gene transfer mediated by SeV vector. Sections of rat abdominal aorta (A, B, C, and D) and rat carotid arteries (E and F) transfected with NLS-lacZ/SeV were shown. For gene transfer into the abdominal aorta, rats were subjected to ventrotomy. After the arteries and veins were ablated from abdominal aorta, the branch was clipped to stop blood flow, and protease was injected using the needle for insulin injection. After 5 min, the aorta was washed extensively with saline and injected with 25 µl of NLS-lacZ/SeV (1x 10⁹ pfu/ml). After 2 min, blood flow was reestablished. After 3 days, the infected vessels were dissected, fixed, and stained using the β-galactosidase staining kit. For gene transfer into rat carotids , the arteria carotis communis were transfected by similar procedures as described above, except that, in F, the carotids were washed with saline and then administered with protease and vector simultaneously by injecting 10 µl of 1:1 mixture of collagenase type 4 solution (final 500 unit/ml) and NLS-lacZ/SeV solution (final 1x 10⁹ pfu/ml). After 5 min, blood flow was reestablished. After 3 days, the infected vessels were dissected, fixed and stained using the β-galactosidase staining kit. In the artery without protease treatment, SeV transfection was only found into the vascular endothelial cells, whereas, in the arteries pretreated with such enzymes, the vector was transfected into media. The medial gene transfer was also observed in the artery treated with protease and vector simultaneously.
   "untreated": without protease treatment, "MMP9": treated with activated MMP9, "plasmin": treated with plasmin, "MMP2": treated with activated MMP2, and "collagenase": treated with collagenase type 4.

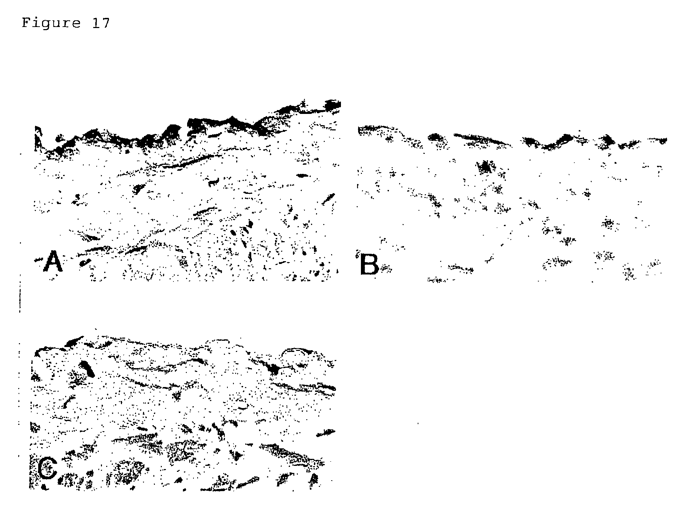

Figure 17 shows photographs of human great saphenous vein subjected to *ex vivo* SeV vector-mediated gene transfer by protease treatment. Human great saphenous veins obtained from patients of lower limb varix on surgery were used to examine the effect of protease treatment on ex vivo SeV vector-mediated gene transfer into neointima. Sections of the vein without protease treatment (A), the veins simultaneously treated with MMP2 and NLS-lacZ/SeV (1x 10⁹ pfu/ml) (B), or the veins simultaneously treated with plasmin and NLS-lacZ/SeV (1x 10⁹ pfu/ml) (C) are shown. Treatment with MMP2 or plasmin enabled transfection into neointima underneath basal lamina.
   "untreated": without protease treatment, "MMP2": treated with activated MMP2, and "plasmin": treated with plasmin.

### Best Mode for Carrying out the Invention

The present invention is illustrated in detail below with reference to examples, but it is not to be construed as being limited thereto. All the references (including references, patents, and published patent applications) cited herein are incorporated by reference. The methods used in the Examples are described below.

### i) Cells and in vitro gene transfer

Bovine aorta smooth muscle cells (BSMC) were prepared from bovine aorta as described previously (Yonemitsu Y. et al., Lab. Invest. 75:313-323 (1996); Yonemitsu Y. et al., Biochem. Biophys. Res. Commun. 231:447-451 (1997); Yonemitsu Y. et al., Circ. Res. 82:147-156 (1998)). COS7 cells, which are African green monkey kidney cells transformed with simian virus 40 large T antigen, and human embryonic kidney cells (HEK293) were obtained from ATCC (American Type Culture Collection). Cells were cultured in plastic dishes containing Dulbecco's modified Eagle medium (DMEM) supplemented with 10% fetal bovine serum (FBS). BSMC at passages 6 to 8 were used in the experiments. In all the experiments , transfection was performed by adding a solution of various concentrations of vector to the culture medium. Except in Figure 4, each reporter gene was assayed at 48 hr after transfection unless otherwise specified. Details of each experiment were described herein and legend in the figures.

### ii) Luciferase assay and luminescent β-galactosidase assay of cultured cells.

Forty-eight hours after transfection, cells were washed twice with phosphate-buffered saline (PBS: 137 mM NaCl, 3 mM KCl, 8 mM Na₂HPO₄, and 1 mM KH₂PO₄, pH 7.2), and treated with 200 µl of 1x Cell Culture Lysis Reagent (Promega, Madison, MA). The cell lysates were transferred into 1.5 ml Eppendorf tubes, and centrifuged at 15,000 rpm to remove cell debris. In luciferase assay, 20 µl of the supernatant was added to 100 µl of luciferase assay buffer (Promega), and the relative activity of luciferase was measured on a luminometer (Model LB9507, EG&G Berthold). In luminescent β-galactosidase assay, 20 µl of the supernatant was added to 200 µl of β-galactosidase reaction buffer (Clontech Inc., Palo Alto, CA), and the relative activity of luciferase was measured on a luminometer. Concentration of the proteins was determined by Bradford method using a commercial protein assay system (Bio-Rad Laboratories Ltd., Hertfordshire, UK), and data were expressed as RLU (relative light unit)/mg protein. Representative results were obtained by repeating experiments at least three times.

### iii) X-Gal histochemistry of cultured cells

Forty-eight hours after transfection, cells were washed twice with PBS, and fixed with 2% formaldehyde for 10 min. Cells were washed twice by immersing in PBS containing 0.1% Triton X-100, then X-Gal solution (0.2% 5-bromo-4-chloro-3-indolyl-β-D-galactoside, 1 mM K₄(CN)₆, 3.3 mM K₃(CN)₆, 60 mM Na₂HPO₄, and 40 mM NaH₂PO₄) was added to each well, and the mixture was left at room temperature for 1 hr. The staining reaction was terminated by immersing cells in PBS, and cells were examined under phase contrast microscope.

### iv) Gene transfer into human saphenous vein and cultured organs

Human great saphenous veins were prepared freshly from patients during ablation of the varix in the lower limb, and subjected to one of the following two different gene transfer methods at random. Twenty veins were collected (male: 6, female: 14; age: 22 to 82, average 57.4), from which two veins having obvious phlebitis were excluded. The veins obtained were cut into several sections and used for the following experiments.

### 1. Simple floating method:

Eighty-five sections were used in total (56 for luciferase assay and 29 for X-gal histochemistry). Veins were washed four times with PBS, and further washed three times with DMEM supplemented with 10% FBS to remove blood. The veins were sliced into sections of 5 to 10 mm length, and immersed in fresh medium containing various concentrations of vector. At an appropriate time point, the veins were washed by immersing in culture medium, transferred into 6-well plates, and cultured (organ culture).

### 2. Gene transfer into lumen:

Total 71 sections were used. Veins were cut at 3 to 4 cm intervals to prepare segments. To ensure that valve does not interfere with injection, the external cylinder of the 24G needle was inserted into the segments from the distal end, and tied with 2-0 silk thread. The distal end was fixed by vessel clamps. Two ml of vector solution containing various concentrations of vector was injected into the luminal space with continuous positive pressure (measured by hemodynamometer) at 0 (mock exposure: n=24), 150 mmHg (n=11), 300 mmHg (n=9), or 760 mmHg (n=5) , and incubated for 10 min. Some of the segments were injured on the endothelium by scraping with the 4 Fr Forgarty catheter (0 mmHg: n=7, 150 mmHg: n=6, 300 mmHg: n=9). After 48-hr organ culture, the segments were subjected to an appropriate reporter assay.

### v) X-Gal histochemistry of human vein

Forty-eight hours after transfection, the segments of veins were immersed in and washed with PBS, and fixed with 2% formaldehyde plus 0.25% glutaraldehyde for 10 min. Then, the segments were immersed in and washed with PBS containing 0.1% Triton X-100, and incubated in X-Gal solution for 3 hr on a reciprocal shaker . X-gal stained samples were further fixed with phosphate-buffered formalin, and examined under stereomicroscope. Then, the samples were cut at about 5 mm intervals to prepare smaller segments , which were embedded in paraffin (1 to 6 segments/tissue) to prepare sections of 3 µm in thickness. Thus obtained sections were stained with nuclear fast red or ordinary hematoxylin-eosin staining, and examined under light microscope. The numbers of examined tissue sections are shown in Table 1.

### vi) Luciferase assay of human vein

Forty-eight hours after transfection, the segments of veins were immersed in and washed with PBS, and cut into small pieces with scissors in 500 µl of 1x Cell Culture Lysis buffer. Cell lysates were centrifuged at 15,000 rpm for 5 min at 4°C, and 20 µl of the supernatant was subjected to the luciferase assay using a luminometer as described above.

### vii) Antibodies and immunohistochemistry

Primary antibodies used in the Examples are as follows; a monoclonal antibody against CD34 (Dakopatts) for endothelial cells, and a monoclonal antibody against HHF35 (Enzo Co., Farmingdate, NY) for vascular smooth muscle cells. Immunohistochemistry was performed by the standard avidin-biotin complex method (Hsu S.M. et al., J. Histochem. Cytochem. 29:577-580 (1981a)) with minor modifications. Briefly, thin sections of veins that had been stained with X-Gal and embedded in paraffin were depleted of paraffin completely, and treated with normal rabbit serum (1:10 dilution) for 30 min to block non-specific binding. The sections were incubated with primary antibodies (or with non-sensitized mouse IgG as a negative control) at 4°C overnight. The sections were treated with methanol solution containing 0.3% H₂O₂ for 30 min to block endogenous peroxidase activity, and then incubated with biotin-conjugated rabbit anti-mouse IgG+IgA+IgM antibodies (Histofine SAB-PO(M) kit; Nichirei Co. Ltd., Tokyo, Japan) for 30 min. The sections were washed extensively with PBS, then incubated with avidin-biotin-conjugated horse radish peroxidase complex (Nichirei Co Ltd.), and finally developed by incubating with 0.03% H₂O₂ and 0.1% diaminobenzidinetetrahydrochloride (Merck, Darmastadt, Germany).

### viii) Statistical analysis

All data were expressed as means ± standard deviation (S.D.). Data were analyzed by Mann-Whitney U-test, and the null hypothesis was rejected at p < 0.05.

### [Example 1] Construction and reconstitution of recombinant Sendai virus vectors

Recombinant SeV was constructed as previously described (Kato A. et al., Genes Cells 1:569-579 (1996); Kato A. et al., EMBO J. 16:578-587 (1997); Yu D. et al., Genes Cells 2:457-466 (1997); Hasan M.K. et al., J. Gen. Virol. 78:2813-2820 (1997); Sakai Y. et al., FEBS Lett. 456:221-226 (1999)). First, an 18 bp spacer sequence containing the NotI site (5'-(G)-CGGCCGCAGATCTTCACG-3'; SEQ ID NO: 3) was inserted into the region between the 5'-untranslated region and the initiation codon of the gene encoding nuclear (N-) protein. Thus cloned SeV genome also contains the self-cleaving ribozyme site derived from the antigenomic strand of hepatitis virus delta (Kato A. et al., Genes Cells 1:569-579 (1996)). Full length cDNA encoding luciferase and *E*.*coli* lacZ gene containing the nuclear localization signal (NLS) were amplified by polymerase chain reaction (PCR) using primers containing the NotI site and a set of SeV E and S signal sequence tags for a foreign gene. The amplified products were inserted into the NotI site of the cloned SeV genome. Total length of a template SeV genome containing a foreign gene was adjusted to be a multiple of hexamer (so called "the rule of six"; Kolakofsky D. et al., J. Virol. 72:891-899 (1998)). The template SeV genome containing foreign gene and plasmids encoding N-, P-, and L-protein (pGEM-N, pGEM-P, and pGEM-L, respectively) were mixed with a commercial cationic lipid to form a complex, and the complex vaccinia virus vT7-3 were co-transfected into CV-1 or LLMCK2 cells (Fuerst T.R. et al., Proc. Natl. Acad. Sci. U.S.A. 83:8122-8126 (1986)). After 40 hr, cells were disrupted by freeze-thawing three times, and injected into the chorioallantoic lumen of 10-day-old embryonated chicken eggs. The resulting recombinant virus was collected, and re-amplified in chicken eggs to get rid of vaccinia virus. The virus titer was determined by measuring hemagglutination activity using chicken erythrocytes (Yonemitsu Y. and Kaneda Y. Hemagglutinating virus of Japan-liposome-mediated gene delivery to vascular cells. Ed. by Baker A.H. Molecular Biology of Vascular Diseases. Methods in Molecular Medicine: Humana Press, pp. 295-306 (1999)). Virus-containing allantoic solution is prepared as a composition containing the recombinant virus vector of the present invention (SeV-NLS-lacZ), and stored at -80°C as a frozen stock until use. A composition containing another recombinant virus vector, SeV-luc (Hasan M.K. et al., J. Gen. Virol. 78:2813-2820 (1997)), was similarly prepared.

### [Example 2] Dose-dependent transfection of SeV in vivo and ex vivo

Logarithmically growing cultures of an established cell line, COS7 cells and human embryonic kidney HEK293 cells, both of which are well known to be easily transfected, as well as BSMC were used to estimate the dose-dependent SeV-mediated gene transfer. Forty-eight hours after SeV-luc transfection, in both COS7 cells and HEK293 cells, luciferase expression was increased in dose-dependent manner, and it reached a plateau at titers higher than MOI=3 (data not shown). Under similar conditions, the luciferase expression in BSMC also increased in dose-dependent manner, and continued to increase even at MOI=100, as shown in Figure 1 (n=6 for each; untreated control: 46,990.57±3,849.08 RLU (relative light unit)/mg; mock transfection where wild-type SeV was used at MOI=100 : 4.3±0.3 (x 10⁴) RLU/mg; SeV-luc at MOI=0.1: 4.1±0.2 (x 10⁴) RLU/mg; SeV-luc at MOI=1.0: 4.5±0.6 (x 10⁹) RLU/mg; SeV-luc at MOI=10: 3.4±0.2 (x 10¹⁰) RLU/mg; and SeV-luc at MOI=100: 9.5±0.7 (x 10¹⁰) RLU/mg). Next, the present inventors evaluated the transfection efficiency using SeV-NLS-luc (n=3 for each, Figure 2). X-Gal histochemistry showed that the frequency of blue-stained cells was increased in dose-dependent manner, and that, at MOI=100, 90% or more BSMC became positive.

Next, the present inventors examined whether the recombinant SeV was capable of being transfected into human vessel wall, using human great saphenous veins that were obtained on surgery. Segments of veins, each 5 to 10 mm in length, were incubated in vector solution containing SeV-luc at different concentrations for 1 hr, washed 4 times by immersing in medium, and then immediately cultured in fresh medium (n=6 for each) . After 48 hr, luciferase activity was measured. Data in Figure 3 show that the luciferase activity increased clearly in dose-dependent manner as in BSMC (n=6 for each; untreated control: 1.7±0.4 (x 10⁴) RLU/mg; SeV-luc (10⁷ pfu) : 1.2±0.2 (x 10⁸) RLU/mg; SeV-luc (10⁸ pfu): 1.2±1.0 (x 10⁹) RLU/mg; SeV-luc (10⁹ pfu): 2.7±0.8 (x 10⁹) RLU/mg).

### [Example 3] Time-dependent gene expression level after SeV-mediated gene transfer in vitro

To get information on time-dependent expression of a foreign gene transfected by SeV, the present inventors evaluated the luciferase activity in BSMC at certain time points after treatment with SeV-luc at MOI=10 (n=6 for each point). As shown in Figure 4, significant luciferase activity was already detected just 1 hr after transfection, and the expression level increased logarithmically in time-dependent manner (mock transfection: 4.6±0.4 (x 10⁴) RLU/mg; 1 hr after transfection: 2.3±0.3 (x 10⁶) RLU/mg; 6 hr after transfection: 1.9±0.3 (x 10⁷) RLU/mg; 12 hr after transfection: 1.9±0.1 (x 10⁸) RLU/mg; and 24 hr after transfection: 2.9±0.5 (x 10¹⁰) RLU/mg). The luciferase activity reached a plateau 2 to 3 days after transfection (2 days after transfection: 2.1+0.2 (x 10¹¹) RLU/mg; and 3 days after transfection: 3.7±0.2 (x 10¹¹) RLU/mg). Accordingly, the assay was usually performed 2 days after transfection in the following Examples .

### [Example 4] Effect of SeV-mediated gene transfection on cell proliferation

Next, the present inventors examined the effect of SeV-mediated gene transfection on the proliferation of BMSC to evaluate the cytotoxicity of the vector against BSMC. As shown in Figure 5, the proliferation of BSMC was not inhibited by the infection of wild-type SeV up to MOI=100. However, on day 8 at MOI=1,000, the cell proliferation was clearly inhibited (p<0.01).

### [Example 5] Time course of foreign gene expression

Paramyxoviridae including SeV replicate their genome and express there genes in the host cytoplasm. This suggests stable expression of reporter genes in the daughter cells . However, the expression level of therapeutic genes could be affected by genome having DI (defective interfering) effect or by possibility of accumulation of spontaneous mutations (Calain P. et al., Virology 212:163-173 (1995)). To test this, the present inventors evaluated the time course of reporter gene expression after SeV-luc-mediated gene transfer using proliferating cells or quiescent cells, in a series of the following experiments.

First, BSMCs growing logarithmically in 75 mm² flasks were infected with SeV-luc at MOI=0.1, 1, and 10. At the indicated time points, three quarters of the cells were subjected to luciferase assay, and the rest of them were subjected to passage culture. For the three different dosages, the luciferase expression was sustained at high level until day 37 and relatively stable (Figure 6a) . The experiment was repeated three times, and similar results were obtained.

Next, the present inventors evaluated the time course of reporter gene expression using quiescent BSMC, as follows. Cells were rendered to be confluent (dense), and left for additional 2 days or longer to stop cell cycle. More than 95% of BSMCs were made to be in the G1/G0 phase using FACScan (data not shown) . The quiescent BSMCs were exposed to SeV-luc at MOI=0.1, 1, and 10 for 1 hr, and subjected to luciferase assay at the indicated time points (n=6 for each, Figure 6b). The expression of the foreign gene reached the maximum level on day 2 (1.1±0.4 x 10¹¹ RLU/mg) and was gradually falling down as time passed. The luciferase activity reached the lowest level on day 14 (2.1±0.8 x 10¹⁰ RLU/mg). Then, the luciferase activity began increasing gradually, and kept increasing at least until day 28. These results indicate that the expression of a gene transfected by SeV can be sustained at relatively high level over at least 1 month.

### [Example 6] Effect of vector exposure time on SeV-mediated gene transfer into BSMC and human saphenous vein

The current vectors used for gene transfer require relatively long exposure time, which is considered as one of the major limitations on the vectors when they are used in clinical applications. To check this in the SeV-mediated gene transfer system, the present inventors examined the dependency of the transfection efficiency on exposure time using quiescent BSMC and human saphenous vein.

For BSMC, cells were plated in 6-well plates, rendered to be confluent, and left for additional 2 days. It is confirmed using FACScan that more than 95% of BSMCs were in the G0/G1 phase (data not shown) . SeV-luc (MOI=10) was added to the each wells. The medium was removed after 1, 2, 5, 10, 30, 60, and 180 min, and 24 and 48 hr, and cells were washed twice with fresh medium. Then, 2 ml of DMEM containing 10% FBS was added to the wells. Forty-eight hours after transfection, cells were subjected to luciferase assay as described above.

Interestingly, as shown in Figure 7a, SeV-mediated luciferase gene expression was hardly affected by the time for interaction between the vector and cells: 1 min exposure was enough to achieve similar level of luciferase activity to that obtained by 48 hr exposure (n=6 for each).

The above findings were representatively observed in human saphenous veins (Figure 7b) as well, and similar findings were also observed when BSMCs were transfected with SeV-NLS-lacZ. As shown in Figure 7b, treatment for just 1 min with vector solution was enough to achieve a similar level of luciferase activity to that obtained by 48 hr treatment. These results indicate that the time for interaction between the vector and cells is not critical for SeV-mediated gene transfer.

In contrast, the transfection activity of a lacZ-encoding adenovirus vector was dependent on the incubation time under similar conditions, as reported previously (Guzman R.J. et al., Circulation 88:2838-2848 (1993)) (Figure 8).

### [Example 7] Efficiency of transfection into human saphenous vein.

To identify the exact cell types that are transfected by SeV-mediated gene transfer in human blood vessel, human great saphenous veins were obtained from patients upon ablation of the varix. The merits of their use are (1) that one can use diseased human blood vessels containing various degrees of myofibrillar neointima, (2) that it is easy to obtain vessels in large quantity, and (3) that one can avoid bacterial contamination, which happens frequently. Conversely, the disadvantages are (1) that ECs can be easily lost by manipulations, and (2) that such veins tend to have severe fibrosis in the vessel wall and reduced number of medial cells.

First, the present inventors evaluated the gene transfer through the lumen using different pressures for injection. Two milliliter of SeV-NLS-lacZ (5x 10⁸ pfu) was injected into 3- to 4-cm vein segments with pressure 0, 150, 300, and 760 mmHg for 10 min (n=6 for each). The vector solution was also injected using the same pressures into the vessels injured by the balloon catheters (n=6 for each). As a control, wild-type SeV was injected (n=6). The gene expression was estimated by X-Gal staining after 48 hr.

The viability of vein cells was evaluated by counter staining of the nuclei using Hematoxilin (Table 1). In the group that had been treated with 760 mmHg, the numbers of CD34 positive EC and HHF35 positive VSMC were clearly reduced in all five segments; therefore, the group was excluded from further evaluations. In the group injured with the balloon catheters, the luminal surface was found severely injured; therefore, the ECs of the lumen were not evaluated. For other groups, there was no significant difference in the average cell number among groups; therefore, they were evaluated by X-Gal staining.

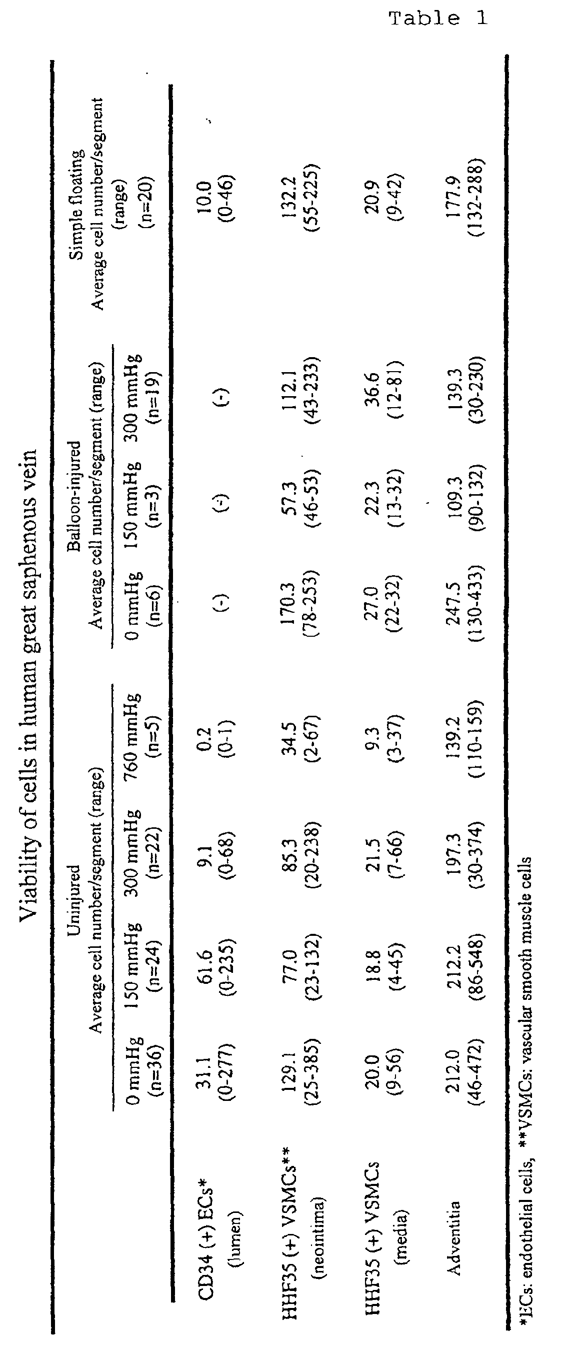

Examination of the vessels exposed to SeV-NLS-lac Z with different injection pressures revealed that blue spots intensely stained were distributed at high frequency on the luminal surface and in adventitia (Figures 9a and b). Blue spots were not found in the vessel exposed to the wild-type SeV (Figure 9c) . Using histological tests, most of the blue cells were identified as ECs on the luminal surface or those of vasa vasora, and as fibroblasts in adventitia (Figures 9d and e). This was confirmed by immunohistochemistry (data not shown).

In the balloon-injured vessel, the number of blue spots on the luminal surface was greatly reduced (Figure 10a), whereas the number in adventitia including vasa vasora was similar to that in uninjured vessels (Figure 10b, arrowhead). The results were confirmed by histochemistry; cells containing blue nuclei were sparsely distributed in vessels having thin neointima (Figure 10c, arrowhead), whereas few of them were observed in vessels having thick neointima, except scattered capillary ECs (Figures 10d and e, arrow) . Certain area of broken neointima was found to contain medial cells positive for X-Gal at relatively high frequency, which suggests that the transfection efficiency of SeV vector into vascular smooth muscle cells may not be low (Figures 11f and g, arrow). These results indicate that neointima substantially interferes with SeV-mediated gene transfer into human blood vessels whereas ECs do not.

Sections were further subjected to immunohistochemistry to determine the ratio of X-gal positive cells in intima, media, and adventitia (Table 2). At all injection pressures, the efficiency of SeV-mediated transfection into the uninjured vein was almost equal to that obtained by simple floating method, whereas the number of transfected cells was slightly increased at 300 mmHg in neointima using the injured vein. Additionally, when neointima was torn, there was obvious increase in the number of X-Gal positive cells in media and adventitia at 300 mmHg. This is consistent with the histological data shown in Figures 11f and g. The efficiency of transfection into neointima was still low in torn samples, which suggests that the vector permeability into neointima area is low.

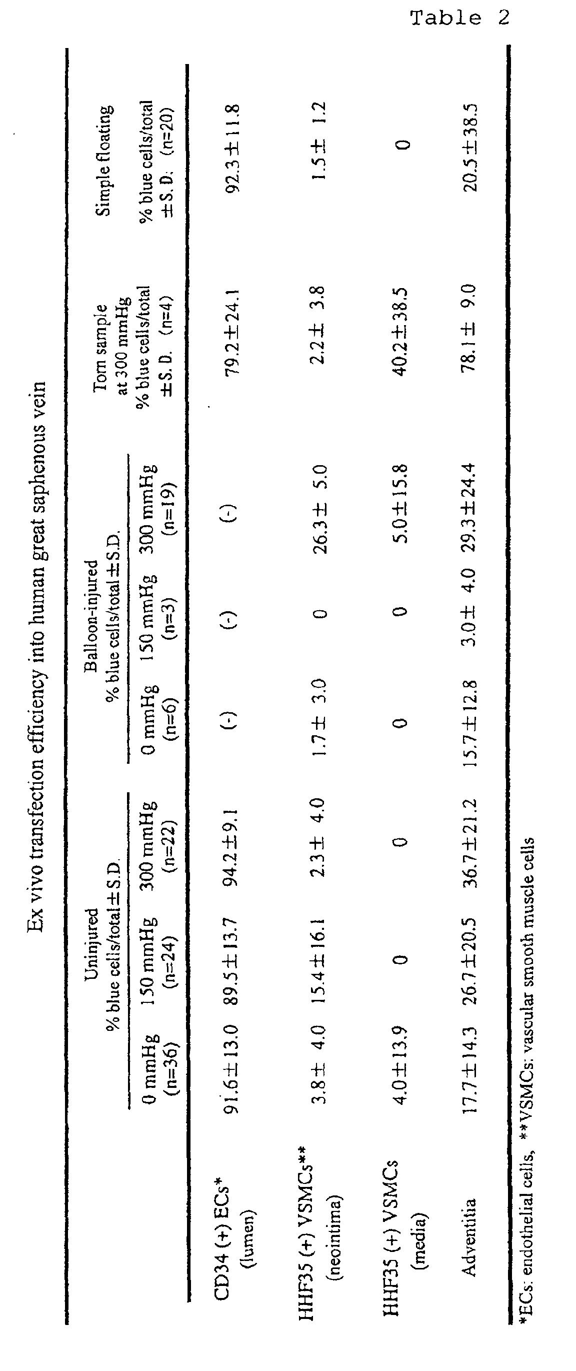

### [Example 8] In vivo gene transfer into rabbit carotid artery

Male Japanese White rabbits (2.5 to 3.5 kg in weight) anesthetized with pentobarbital were cut at the neck by midline incision, and the carotid arteries were exposed. Sheath of adventitia surrounding the media was picked up by fine forceps, and appropriate amount of SeV-NLS-lacZ (5x 10⁷ pfu) (usually approximately 500 µl) was injected using 26-gauge needle, with media kept uninjured. Then, the incision was sewed up together.

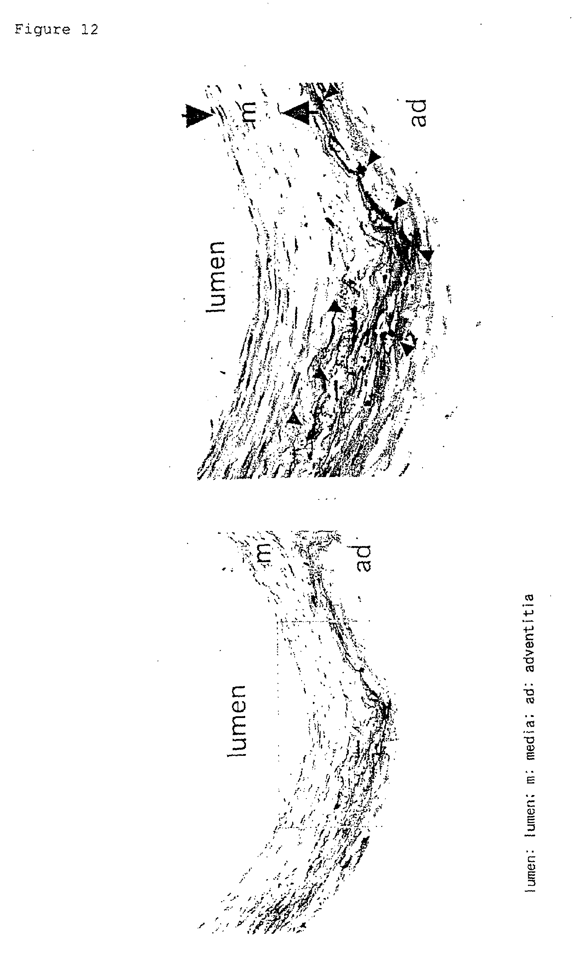

After 2 days , rabbits were anesthetized again with pentobarbital, the incision was opened, and 18-gauge elaster was inserted through the external carotid . After the lumen was washed with saline containing heparin, the arteries were cut off and fixed with fixative solution (2% paraformaldehyde plus 0.25% glutaraldehyde) for 10 min. The vessels were washed extensively with PBS, immersed in X-Gal solution, and incubated at room temperature for 3 hr on a shaker. Then, the vessel walls were washed with a large volume of PBS, fixed again with neutral formalin, and sectioned. As a result, the nuclei of adventitia cells were stained blue, indicating that SeV-mediated gene transfer into vascular cells was effective *in vivo* as well (Figure 12).

By similar methods, it is possible to transfer a gene into the lower limb bypass grafted vessel wall, coronary aorta in heart, or so on.

Injection into the lumen using positive pressure may be performed as follows. For rabbits, Japanese White rabbits anesthetized with pentobarbital plus ketamin were cut at the neck by midline incision, and the right common carotid artery, internal carotid artery, and external carotid artery were exposed. The branch vessels from the common carotid artery were bent up, and the common carotid artery and internal carotid artery were clamped at the end closer to the heart and the end closer to the head, respectively. A small hole was made on the external carotid artery wall using small scissors, and the 18-gauge double lumen catheter was kept against internal carotid artery. One end of the catheter was opened, and approximately 5 cc of buffer was injected through the other end to wash out blood components completely. Then, one end was closed and the other was connected with hemodynamometer. Virus solution was injected through trivalve junction linked to the tube connecting with hemodynamometer, and the lumen was filled. The end was closed, arbitrary pressure (normally 150 mmHg) was applied by hemodynamometer, and the artery was left for 10 min. The virus solution was recovered, the catheters were removed, and the external carotid was tied. The common carotid artery and internal carotid artery were opened to reestablish blood flow.

### [Example 9] Ex vivo gene transfer of SeV vector into rat thoracic artery

The efficiency of SeV-mediated gene transfer into rat artery was examined using rat thoracic arteries. Wister rats (Charles River) , 9- to 12-week-old male, anesthetized with Nembutal were subjected to ventrotomy, and the thoracic aorta was dissected. The blood vessels were washed with Hanks solution (GIBCO BRL), and tubular portions cut vertically into laminar pieces. The laminae were further cut into approximately 4 x 5 mm laminae. The laminae were washed with Hanks solution, and immersed for 10 min in Hanks solution which contained GFP/SeV expressing GFP or SeV-NLS-lacZ at the indicated concentrations. The samples were washed twice with Hanks solution, and cultured in DMEM (GIBCO BRL) containing 10% FBS (GIBCO BRL) in an incubator at 5% CO₂. The next day, the medium was replaced with fresh one. After an additional day, the samples were fixed and stained for 3 hr using the β-gal staining kit (Invitrogen). After cell number was counted, cells were post-fixed, and frozen sections and paraffin sections were prepared.

To prepare frozen sections, the laminar samples were embedded with OCT compound (Tissue-Tek) after staining with the β-gal staining kit (Invitrogen). Then, 6 µm sections were prepared using a cryostat, and counter stained using nucleo fastred (Wako, 142-04331). Toprepare paraffin sections, the laminar samples were stained with the β-gal staining kit and post-fixed with 10% formalin . Then, the samples were dehydrated with ethanol, permeabilized, embedded with paraffin, and cut into 4 µm sections with a microtome.

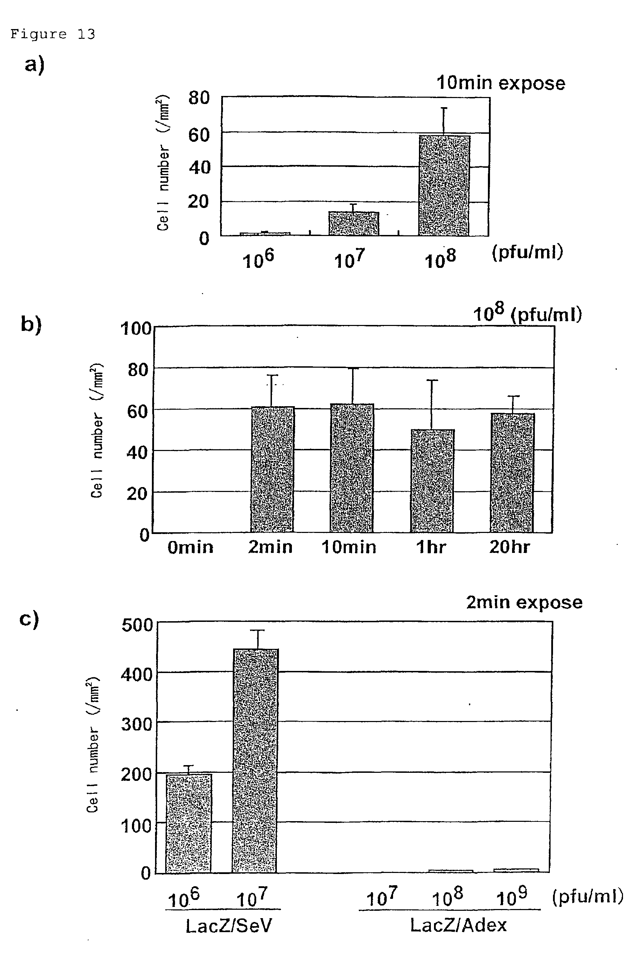

As was in the cultured vascular cells and in human great saphenous veins described in above Examples, the transfection efficiency of the vectors was increased in dose-dependent manner (Figure 13a) , and reached a plateau with 2 min exposure (Figure 13b). The SeV vector achieved high transfection efficiency at lower concentrations as compared with adenovirus vector (Figure 13c).

### [Example 10] Improved infectivity and transfection into media by protease treatment

To expand the potential use of the Paramyxovirus vector, protease pretreatment was applied to digest extracellular matrix (ECM) , which interferes with the vector transfection, especially into macrophages and smooth muscle cells, which are locating in the more inner area than endothelial cells. Macrophages and smooth muscle cells are the main factors causing post PTCA restenosis and arteriosclerosis. To determine which type of protease promotes transfection into media, ECMs were treated with various proteases for 5 min before SeV vector was transfected. Since the transfer of enzyme is restricted to the vascular endothelial cells, protease that are capable of digesting basal lamina, and those used in clinical applications to blood vessels were mainly selected. The rat thoracic aorta was isolated and cut into small pieces as in Example 9, and the segments were incubated at 37°C for 5 min in Hanks solution or 10% FBS (GIBCO BRL)-containing Hanks solution supplemented with the following protease: collagenase type 4 (ICN), 500 unit/ml; urokinase (COSMO BIO), 500 unit/ml; tPA (Calbiochem), 500 unit/ml; elastase (COSMO BIO), 500 unit/ml; plasmin (COSMO BIO) , 50 µg/ml; active-MMP2 (COSMO BIO), 100 ng/ml; active-MMP9 (COSMO BIO) , 100 ng/ml. Solutions were pre-equilibrated to 37°C by warming at 37°C for 10 min. The segments were washed twice with Hanks solution, and immersed in Hanks solution containing GFP/SeV or SeV-NLS-lacZ at the indicated concentrations for 2 min. The segments were washed twice with Hanks solution, and cultured in DMEM (GIBCO BRL) containing 10% FBS (GIBCO BRL)at 5% CO₂ in an incubator. After a day, the media was replaced, and the culture was continued for an additional day. Then, the segments were fixed and stained using the β-gal staining kit (Invitrogen). The staining time was 3 hr. After the number of cells was counted, and the segments were post-fixed, frozen sections and paraffin-embedded sections were prepared as in Example 9.

As shown in Figure 14, the infectivity was increased by 2- to 5-fold when SeV was infected at 1x 10⁷ pfu/ml with collagenase, urokinase, tPA, MMP9, MMP2, or elastase. Figure 15 represents the sections corresponding to the data shown in Figure 14. The examination revealed that pretreatment with collagenase, MMP2, and MMP9 enabled transfection even into the smooth muscle cells in arterial media. These protease are considered to have high activity because they digest basal lamina as a substrate, and particularly, MMP9 and MMP2 can digest a component of elastic fibers, elastin, as well. On the other hand, tPA alone did not achieve medial transfection, but, in combination with serum, tPA enabled transfection into media . This could be because plasminogen in the serum was digested into plasmin.

### [Example 11] In vivo gene transfer of SeV into rat media by protease treatment

The effect of protease treatment on *in vivo* gene transfer was examined by introducing vectors into rat abdominal aorta and carotid artery (Figure 16). Arteries were pretreated with the protease that had promoted ex vivo transfection: collagenase type 4, MMP9, and MMP2, and also plasmin, in place of tPA plus 10%FBS (Figures 16a to d). For the carotid artery, protease and vector were mixed beforehand and administered taking into account of simplification of procedures for clinical application (Figures 16e and f).

For *in* vivo gene transfer into rat abdominal aorta using SeV vector, the rat was anesthetized with Nembutal and subjected to ventrotomy to expose the abdominal aorta. The abdominal aorta was clamped to stop blood flow. To prevent leakage, the branch vessels were also clamped. Twenty-five µl of protease was injected into blood vessels using the insulin needle (TERMO). Proteases and their concentrations to be used were the same as used in the above *ex vivo* experiments. Protease treatment was performed by incubating at 37°C for 5 min with blood flow kept stopped using cotton sticks . The artery was washed with 50 µl of saline using the insulin needle, similarly. Then, 25 µl of SeV-NLS-lacZ (1x 10⁹ pfu/ml) was injected into the vessel, and incubation was continued for 2 min while shielding with cotton sticks. Blood flow was reestablished by unclipping, and the vessel was reconnected. After 3 days, the rat was subjected to ventrotomy again, and the infected vessel was dissected and subjected to lacZ staining.

For *in vivo* gene transfer into rat carotid artery using SeV vector, the rat was anesthetized with Nembutal, and a pillow was put under the lower shoulder to stretch the lower jaw. The common carotid artery, internal carotid artery, and external carotid artery were detached, and all the branching was cut off. The external carotid artery was tied at positions approximately 1 cm apart from the influx sites, and all the blood flow were blocked. The outer cylinder of 24-G elaster was inserted through the external carotid artery, and the lumen was washed extensively with heparin-containing saline. The catheter was fixed by thread. Ten µl of protease solution was injected. After 5 min incubation, the vessel was washed with saline, and then 10 µl of SeV-NLS-lacZ (1x 10⁹ pfu/ml) was injected. After 2 min incubation, the outer cylinder was removed, and the eye of the needle was tied at the center. Blood flow was reestablished and the vessel was reconnected. After 3 days, the rats were perfused with fixative solution (saline containing 1% paraformaldehyde and 0.2% glutaraldehyde) subjected to ventrotomy again, and the injected area was dissected. The samples were subjected to LacZ staining with the β-galactosidase staining kit. For simultaneous administration, solutions separately containing equal amount of protease as described above and SeV were prepared so that a desired final concentration would be obtained for each by mixing them. The solutions were prewarmed at 37°C for 10 min, mixed immediately before transfection, and exposed to the vessel. After 5-min immersion, the vessel was washed twice with Hanks solution. The following procedures were performed as described in Example 9.

In the untreated sample, SeV transfection was only found in endothelial cells, whereas in the samples pretreated with collagen type 4, MMP9, MMP2, or plasmin, the SeV gene transfer was found in the medial layer as well. For gene transfer into rat carotid artery, the common carotid artery was used. In Figure 16f, after washed with saline, the sample was administered with 10 µl of 1:1 mixture of collagenase type 4 (final 500 unit/ml) and SeV-NLS-lacZ (final 1x 10⁹ pfu/ml). After 5 min, blood flow was reestablished. After 3 days, the injected vessels were dissected, fixed, and stained using the β-galactosidase staining kit. In the untreated sample, SeV transfection was only found in endothelial cells, whereas in the samples pretreated with the protease, SeV was transfected into the medial layer as well. Transfection into the medial layer was also achieved by simultaneous administration of protease and vector.

### [Example 12] Ex vivo gene transfer of SeV into human great saphenous vein by protease treatment

*Ex vivo* gene transfer of SeV into neointima with the aid of protease pretreatment was performed using human great saphenous vein, which was provided from patients upon ablation surgery of the varix. Fat was removed from the thus-obtained great saphenous vein, and the vessel was cut into 1 cm x 1 cm laminar pieces and washed with Hanks solution. The sample was treated at 37°C for 5 min with protease-containing Hanks solution that had been prewarmed at 37°C for 10 min. After washed twice with Hanks solution, the sample was immersed in Hanks solution containing SeV-NLS-lacZ (5x 10⁸ pfu/ml) for 2 min. After washed twice with Hanks solution, the sample was cultured in DMEM (GIBCO BRL) containing 10% FBS in an incubator at 5% CO₂. After a day, the culture medium was replaced, and the culture was continued for an additional day before fixation and staining with the β-gal staining kit. The sample was stained for 3 hr. Then, the number of cells was counted, the sample was post-fixed, and paraffin sections was prepared.

As was the case in rat arteries, SeV transfection was only found in endothelial cells in the untreated sample (Figure 17a). In contrast, in the samples treated with MMP2 or plasmin, transfected cells were found in neointima under basal lamina as well (Figures 17b and c). All the proteases used in the experiments in rat were tested, and only MMP2 and plasmin showed significant effect. The reason might be that human great saphenous vein from patients of lower limb varix has an enlarged layer under basal lamina, whose structure is quite different from those of rat arteries. Additionally, it has different compositions of extracellular matrix and contains fibrin fibers. Therefore, it is reasonable that plasmin, which can digest both basal lamina and fibrin, exhibited significant effect.

### Industrial Applicability

The efficiency of transfection into the blood vessel has not been at satisfactory level using conventional vectors. The use of a Paramyxovirus vector of the present invention has enabled efficiently transfecting genes into the vascular cells in short time. Thereby, the present invention has provided a basic technology for gene therapies such as those targeted at vascular cells.

## Claims

1. A method for transferring a nucleic acid into a vascular cell, said method comprising a step of contacting a recombinant Paramyxovirus vector or a cell comprising said vector with said vascular cell.

2. A method for transferring a nucleic acid into a vascular cell, said method comprising performing the following steps (a) and (b) sequentially or simultaneously:
(a) treating a tissue comprising a vascular cell with a protease, and
(b) contacting a recombinant Paramyxovirus vector or a cell comprising said vector with said vascular cell.

3. The method according to claim 2, wherein said protease is selected from the group consisting of collagenase, urokinase, elastase, tissue plasminogen activator, plasmin, and matrix metalloproteinases.

4. The method according to any one of claims 1 to 3, wherein said nucleic acid contained in the recombinant Paramyxovirus vector comprises a foreign gene.

5. The method according to any one of claims 1 to 4, wherein said vascular cell is selected from the group consisting of an endothelial cell in a vascular lumen, an endothelial cell in a vasa vasorum (vessel wall nutrient vessel) , a vascular smooth muscle cell in vascular media, and an adventitial cell.

6. The method according to any one of claims 1 to 5, wherein said Paramyxovirus is Sendai virus.

7. A recombinant Paramyxovirus vector that transfers a nucleic acid into a vascular cell.

8. The vector according to claim 7, wherein said Paramyxovirus is Sendai virus.

9. The vector according to claim 7 or 8, wherein said nucleic acid contained in the recombinant Paramyxovirus vector comprises a foreign gene.

10. The vector according to any one of claims 7 to 9, wherein said vascular cell is selected from the group consisting of an endothelial cell in a vascular lumen, an endothelial cell in a vasa vasorum (vessel wall nutrient vessel) , a vascular smooth muscle cell in vascular media, and an adventitial cell.

11. A composition for gene transfer into a vascular cell, said composition comprising the recombinant Paramyxovirus vector according to any one of claims 7 to 10.

12. A kit for gene transfer into a vascular cell, said kit comprising the recombinant Paramyxovirus vector according to any one of claims 7 to 10 and a protease.

13. The kit according to claim 12, wherein said protease is selected from the group consisting of collagenase, urokinase, elastase, tissue plasminogen activator, plasmin, and matrix metalloproteinases.
